# FASCICULE DE BREVET EUROPEEN

(11) **EP 1 682 124 B1**
(45) Date de publication et mention de la délivrance du brevet: **19.12.2007**
(21) Numéro de dépôt: 04805330.0
(22) Date de dépôt: 28.10.2004
(51) Int. Cl.: A61K 31/4178, A61K 47/38, A61K 47/08, A61K 47/02, A61K 47/10, A61P 25/16, A61P 25/18, A61P 25/24

(54) **COMPOSITIONS PHARMACEUTIQUES A BASE DE SEL D'IDAZOXAN OU D´UN DE SES POLYMORPHES**
PHARMAZEUTISCHE ZUSAMMENSETZUNG AUF BASIS EINES IDAZOXAN-SALZES ODER EINES SEINER POLYMORPHEN
PHARMACEUTICAL COMPOSITIONS BASED ON IDAZOXAN SALT OR ONE OF ITS POLYMORPHS

(30) Priorité: 28.10.2003 FR 0312626
(43) Date de publication de la demande: 26.07.2006
(73) Titulaire: PIERRE FABRE MEDICAMENT, 92100 Boulogne-Billancourt (FR)
(72) Inventeur: BOUGARET, Joel, F-31460 Caraman (FR); AVAN, Jean-Louis, F-31290 Villefranche de Lauragais (FR); SEGONDS, Roland, F-31400 Toulouse (FR)
(74) Mandataire: Ahner, Francis
(86) Numéro de dépôt international: PCT/FR2004/002773
(87) Numéro de publication internationale: WO 2005/041956

(56) Documents cités:
- WO-A-01/39740
- US-A- 4 818 764
- US-A- 5 492 907
- SCATTON B ET AL: "PHARMACOLOGICAL AND MOLECULAR TARGETS IN THE SEARCH FOR NOVEL ANTIPSYCHOTICS" BEHAVIOURAL PHARMACOLOGY, RAPID SCIENCE, PUBLISHERS, GB, vol. 11, no. 3/4, 2000, pages 243-256, XP008012092 ISSN: 0955-8810

## Description

La présente invention concerne l'industrie pharmaceutique et est relative à la formulation galénique de principe actif. Plus particulièrement, l'invention concerne une formulation stable et adaptée à l'industrialisation de comprimés de sel d'idazoxan, non pelliculés dosés entre 5 et 100 mg de principe actif et ayant une libération conventionnelle. Ces comprimés se présentent dans un conditionnement étanche à la vapeur d'eau. De préférence le sel d'idazoxan utilisé est un polymorphe du chlorhydrate d'idazoxan.

L'idazoxan est connu pour ses propriétés antagonistes sur les récepteurs alpha₂ adrénergiques. Ce composé est décrit dans le brevet EP 033 655 par sa structure chimique, son procédé de synthèse, certaines formulations pharmaceutiques, et son application thérapeutique en tant que médicament antidépresseur. L'idazoxan a été étudié en clinique humaine dans le traitement de la dépression à des doses variant entre 5 et 40 mg, trois fois par jour, sur quatre semaines et a montré une amélioration significative sur l'échelle de Hamilton contre Placebo (Drug of the future, 10, n°9, 782, 1985). Différentes études ont également été menées sur des singes ou des rats pour évaluer l'action des différents composés sur des symptômes analogues à ceux de la maladie de Parkinson, tels que les symptômes induits par la réserpine chez le rat (F.C. Colpaert, Neuropharmacologie, 26, 1431, 1987) ou par la neurotoxine MPTP (F.C. Colpaert et al, Brin. Res. Bul., 26, 627, 1991). Plus spécifiquement, le brevet FR 92 14694 concerne l'utilisation de l'idazoxan et de ses dérivés pour la préparation d'un médicament destiné au traitement de la maladie de Parkinson, sa progression sous sa forme idiopathique et son évolution. La demande FR 96 03674 porte sur des produits contenant du Milnacipran et de l'idazoxan comme préparation pharmaceutique combinée pour une utilisation simultanée, séparée ou étalée dans le temps pour traiter la dépression et ses différentes formes, ainsi que les pathologies dans lesquelles les anti-dépresseurs sont utilisés.

La demande de brevet WO 01/39740 décrit l'utilisation de l'idazoxan combiné avec un antagoniste des récepteurs dopaminergiques D2 pour le traitement de maladies psychotiques sévères. Les compositions selon ce document sont également adaptées pour une administration par voie orale et peuvent contenir de la méthylcellulose, du lactose, du stéarate de magnésium.

Scatton et al. (Behavioural Pharmacology, vol.11, n°3/4, 2000, pages 243-256) suggère que la combinaison d'un antagoniste des récepteurs dopaminergiques D2 avec un antagoniste des récepteurs adrenergiques α₂, tel que l'idazoxan, constituerait un agent antipsychotique particulièrement actif. Il y est également décrit que l'idazoxan potentialise l'effet antipsychotique de la fluphénazine.

Plusieurs problèmes doivent être résolus avant d'envisager une exploitation industrielle à grande échelle de l'idazoxan. Il faut notamment trouver un excipient compatible avec ce principe actif, sélectionner une forme de véhicule adéquate pour garantir sa stabilité, chercher un procédé de fabrication en tenant compte particulièrement du problème d'hygroscopicité de l'idazoxan et de sa stabilité. De plus, le principe actif doit présenter des propriétés physiques adéquates en terme de granulométrie et de comprimabilité. Il est donc nécessaire de choisir un conditionnement étanche adapté, qui protège le principe actif de l'humidité. Ce sont les problèmes encore non résolus que se propose de résoudre la présente invention.

La présente invention concerne une composition pharmaceutique comprenant 5 à 25% d'un sel ou d'hydrate d'idazoxan ou de leurs dérivés, 10 à 40% de cellulose microcristalline, 0,1 à 5% de lubrifiant, et de 0,1 à 0,5% de silice colloïdale et de 25 à 90% de lactose par rapport à la masse totale. De manière préférée, la présente invention concerne une composition pharmaceutique comprenant 5 à 20% d'un sel d'idazoxan ou d'hydrate d'idazoxan, 10 à 40% de cellulose microcristalline, 1 à 5% de lubrifiant, 0,1 à 0,5% de silice colloïdale et de 29,5 à 84,8% de lactose par rapport à la masse totale.

On entend par idazoxan et ses dérivés, le composé de formule générale I: 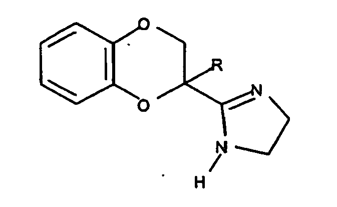 dans laquelle R représente un atome d'hydrogène, un radical alkyl, linéaire ou ramifié en C₁-C₄, et un radical alkoxy, linéaire ou ramifié en C₁-C₄, et ses sels thérapeutiquement acceptables, son racémate, ses isomères optiquement actifs, et ses polymorphes. D'une manière préférentielle, R représente un atome d'hydrogène, un radical méthoxy ou un radical N-propyl. Un aspect de la présente invention concerne donc une formulation stable, telle que définie ci-dessus, comprenant l'idazoxan de formule II: 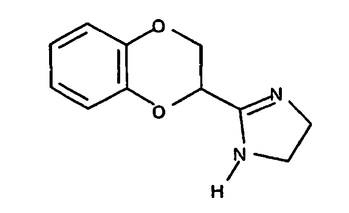 Le chlorhydrate d'idazoxan possède un proton au niveau du centre chiral C2, si bien qu'il existe deux énantiomères potentiels R(-) et S(+). La composition pharmaceutique selon l'invention comprend de préférence le mélange racémique, mais il est envisageable que l'idazoxan de la composition soit enrichi en l'un ou l'autre des énantiomères, voire comprend presque exclusivement l'un ou l'autre des énantiomères, étant donné qu'il est possible de séparer les deux stéréo-isomères au moyen, par exemple, d'une colonne chirale. Les deux énantiomères possèdent des caractéristiques pharmacocinétiques différentes (voir exemple 8), mais même si les deux énantiomères sont séparés, il existe une interconversion d'une forme à l'autre à la fois *in vitro* (dans les conditions physiologiques, pH 7,4, 37°C; voir exemple 8.1) et *in vivo* (voir exemple 8.2). Les formes polymorphes de l'idazoxan sont aussi incluses dans la définition.

Dans un mode préférentiel de réalisation, le sel d'idazoxan est le chlorhydrate d'idazoxan et le lubrifiant est le béhénate de glycérol, le stéarate de magnésium étant incompatible avec le principe actif.

La composition pharmaceutique selon l'invention est particulièrement adaptée à l'industrialisation et est stable. On qualifie une formulation de "industrialisable" ou "adaptée à l'industrialisation", lorsque la formulation permet d'obtenir des lots sur une machine rotative de production tout en satisfais ant aux principaux essais de la pharmacopée européenne en ce qui concerne les comprimés. On entend par stable des comprimés conditionnés générant moins de 1,5% d'impureté de dégradation après un mois à 40°C 75% HR (Humidité Relative) et au plus 2% d'impureté après 2 ans à 25°C 60 HR.

Dans un mode encore plus préféré, le sel d'idazoxan est choisi parmi les polymorphes de forme I, II, III, IV, V et VI ou un mélange d'au moins deux, d'au moins trois, d' au moins 4, 5 ou 6 polymorphes d'idazoxan. La composition pharmaceutique n'est pas destinée à être limitée aux polymorphes de l'idazoxan ou à un mélange de polymorphes de l'idazoxan. D'autres composés à base d'idazoxan adaptés à une utilisation dans cette composition pharmaceutique sont présentés dans les brevets US 2,979,511 et 4,818,764.

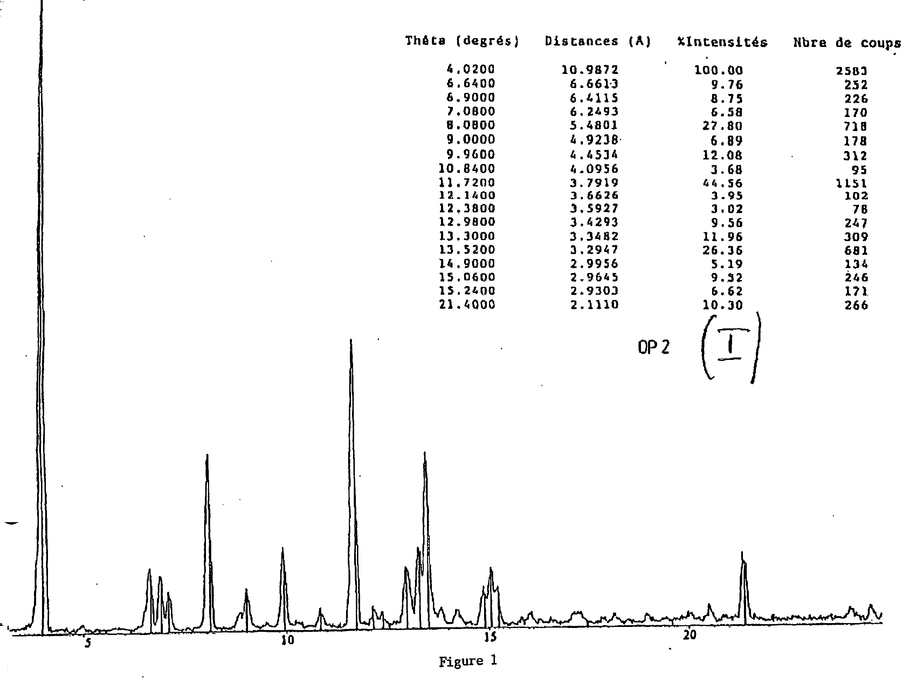

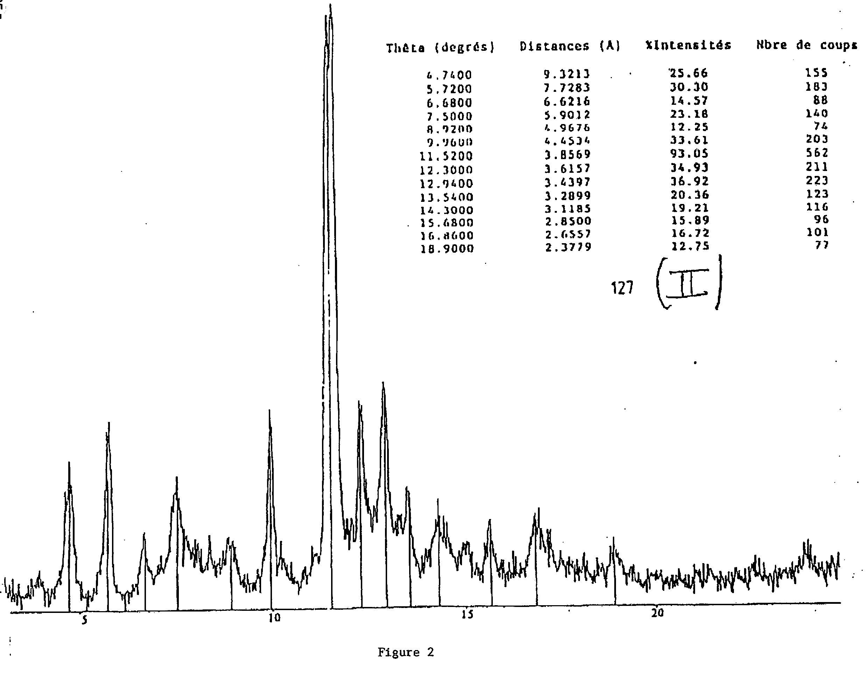

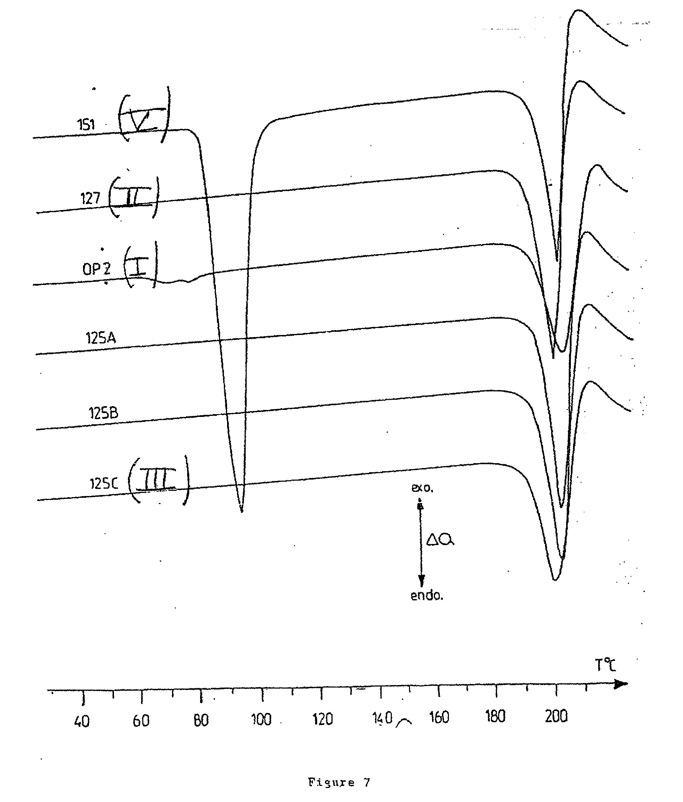

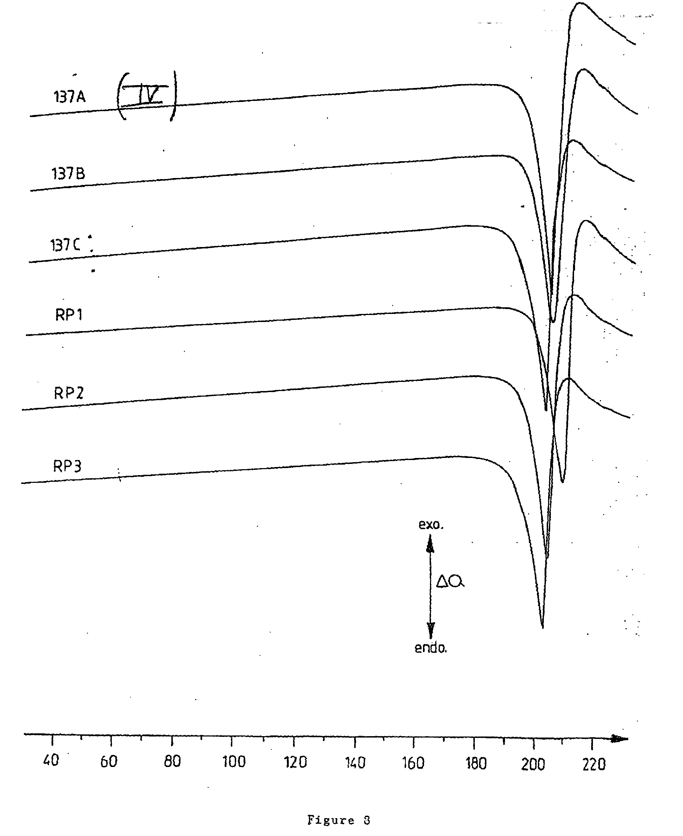

Les polymorphes sont caractérisés par les spectres de diffraction aux rayons X présentés respectivement aux figures 1, 3, 4, 5 et 6 et également caractérisés par les thermogrammes d'analyse thermique différentielle présentés sur les figures 7 et 8 mesurés au moyen d'un Mettler FP 800. Le spectre de diffraction aux rayons X ainsi que le thermogramme d'analyse thermique différentielle mesuré au moyen d'un Mettler FP 800 de l'hydrochlorure d'idazoxan (forme II) sont présentés respectivement sur les figures 2 et 7. Concernant l'analyse thermique différentielles, il convient de remarquer que l'utilisation d'une autre marque ou d'un autre modèle d'appareil d'analyse thermique différentielle (par exemple, Perkins Elmer) peut aboutir à un thermogramme différent. La présente invention est caractérisée par les valeurs du thermogramme présentées ici et obtenues au moyen d'un Mettler FP 800, ainsi que par les valeurs d'un thermogramme équivalent obtenues au moyen d'autres types d'appareils d'analyse thermique différentielle.

C'est également un des objets de la présente invention de protéger ces six formes I, II, III, IV, V et VI en tant que telles.

Le polymorphe de forme I est caractérisé par un spectre de diffraction des rayons X comprenant des pics caractéristiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 et 21,4000 degrés θ. Le polymorphe de forme I est encore caractérisé par un spectre de diffraction des rayons X comprenant des pics caractéristiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 et 21,4000 degrés θ, et manquant d'au moins un pic à environ 4,7400, 5,7200, 8,9200, 16,8600 ou 18,9000 degrés θ. Le polymorphe de forme I est également caractérisé par un thermogramme d'analyse thermique différentielle présentant une valeur maximale unique à environ 207,5 ± 0,2.

Le polymorphe de forme II est caractérisé par un spectre de diffraction de rayons X comprenant des pics caractéristiques à environ 4,7400, 5,7200, 6,6800, 7,5000, 8,9200, 9,9600, 11, 5200, 12, 3000, 12,9400, 13,5400, 14,3000, 15,6800, 16,8600, 18,9000 degré θ. Le polymorphe de forme II est également caractérisé par un thermogramme d'analyse thermique différentielle présentant une valeur maximale unique à environ 203,9 ± 0,4.

Le polymorphe de forme III est caractérisé par un spectre de diffraction des rayons X comprenant des pics caractéristiques à environ 4,0400, 4,7000, 5,7400, 6,6200, 6,9200, 7,4600, 8,0400, 8,7800, 8,9800, 9,9800, 10,8200, 11,4600, 11,6400, 12,3200, 12,9400, 13,5400, 14,2400, 15,0600, 15,6200 et 16,8400 degrés θ. Le polymorphe de forme III est également caractérisé par un thermogramme d'analyse thermique différentielle présentant une valeur maximale unique à environ 203,8 ± 0,5.

Le polymorphe de forme IV est caractérisé par un spectre de diffraction des rayons X comprenant des pics caractéristiques à environ 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 et 18,1000 degrés θ. Le polymorphe de forme IV est encore caractérisé par un spectre de diffraction des rayons X comprenant des pics caractéristiques à environ 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 et 18,1000 degrés θ et manquant d'au moins un pic à environ 6,6800, 13,5400, 15,6800, 16,8600 ou 18,9000 degrés θ. Le polymorphe de forme IV est également caractérisé par un thermogramme d'analyse thermique différentielle présentant une valeur maximale unique à environ 205,3 ± 0,5.

Le polymorphe de forme V est un monohydrate d'hydrochlorure d'idazoxan qui est caractérisé par un spectre de diffraction des rayons X comprenant des pics caractéristiques à environ 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 et 16,7400 degrés θ. Le polymorphe de forme V est encore caractérisé par un spectre de diffraction des rayons X comprenant des pics caractéristiques à environ 5,0400, 5,8400, 7,9400, 9,2800, 9,4400, 10, 1200, 12,0200, 12,5600, 12,.9200, 13,7400, 13,9400, 14,5200, 14,8200, 15,2800, 16,2800 et 16,7400 degrés θ, et manquant d'au moins un pic à environ 4,7400, 6,6800, 7,5000, 8,9200, 11,5200, 14,3000, 15,6800 ou 18,9000 degrés θ. Le polymorphe de monohydrate d'idazoxan de forme V est également caractérisé par un thermogramme d'analyse thermique différentielle présentant une valeur maximale unique à environ 205,6 ± 0,4.

Le polymorphe de forme VI est caractérisé par un spectre de diffraction des rayons X comprenant des pics caractéristiques à environ 5,6150; 6,7350; 7,5350; 9,5250; 10, 3450; 10,6050; 11,0350; 11,2850; 11, 5350: 12,1150; 12,3750; 12,9550; 13, 5150; 13,9950; 14,5250; 14,9350; 15,0450; 15,1950; 16,3450; 17,0450; 17,2850; 17,5750 et 17,8250 degrés θ.

L'invention fournit une méthode de synthèse du polymorphe d'idazoxan des formes I et VI selon un procédé comprenant les étapes suivantes: (i) réaction du catéchol et du 2-chloroacrylonitrile en présence de carbonate de potassium pulvérisé et de bromure de tétrabutylammonium comme catalyseur, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, et (v) recristallisation du chlorhydrate d'idazoxan dans de l'éthanol pour obtenir le polymorphe de forme I. Le procédé pour la synthèse du polymorphe de forme VI comprend en outre les étapes supplémentaires suivantes: (vi) mettre la forme I en suspension dans l'éthanol à 100° sous agitation constante et à température ambiante pendant 1 à 4 jours, (vii) filtrer sous vide, et (viii) sécher dans un four sous vide. Le procédé de synthèse du polymorphe de forme VI peut aussi comprendre en outre, après l'étape (v) de synthèse du polymorphe de forme I les étapes suivantes: (vi') maintenir la forme I en suspension dans l'éthanol à 100° sous constante agitation à haute température, (vii') distiller l'éthanol sous vide jusqu'à évaporation partielle du solvant pour induire la cristallisation, (viii') refroidir la solution à 0°, (ix') filtrer et sécher dans un four.

La présente invention fournit également une méthode de synthèse des polymorphes de l'idazoxan de formes III et IV selon un procédé comprenant les étapes suivantes: (i) réaction du catéchol et du 2-chloroacrylonitrile en présence de carbonate de potassium pulvérisé et de bromure de tétrabutylammonium comme catalyseur, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation de l'hydrochlorure d'idazoxan dans de l'éthanol, et (vi) purification dudit chlorhydrate par recristallisation soit avec de l'éthanol pour obtenir la forme III, soit avec du butanol-1 pour obtenir la forme IV. C'est ainsi que sont obtenus les polymorphes III et IV.

La présente invention fournit également une méthode de synthèse d'un monohydrate d' hydrochlorure d'idazoxan, désigné dans les présentes comme la forme V, selon un procédé comprenant les étapes suivantes: (i) réaction du catéchol et du 2-chloroacrylonitrile en présence de carbonate de potassium pulvérisé et de bromure de tétrabutylammonium comme catalyseur, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol, et (vi) réempâtage en cinq volumes d'un mélange de 80% d'acétone et de 20% d'eau. C'est ainsi qu'est obtenu le monohydrate.

L'invention englobe également les hydrates d'idazoxan et les compositions pharmaceutiques contenant de tels hydrates. Le terme "hydrate" recouvre, mais de façon non limitative, les hémi-hydrates, monohydrates, dihydrates, trihydrates et autres substances du même genre.

La composition selon l'invention est administrée par voie orale, et est donc formulée sous une forme adaptée à l'administration orale, de préférence sous forme solide. Parmi les présentations orales solides adaptées, on peut citer: les comprimés, gélules, granules et autres présentations du même type. Les compositions pharmaceutiques adaptées à l'administration orale comprennent, outre les composés polymorphes de la présente invention, ou leurs mélanges, leurs dérivés, leurs analogues, leurs sels ou hydrates pharmaceutiquement acceptables, un vecteur, un diluant ou un excipient également acceptable sur le plan pharmaceutique. Tout excipient inerte, communément utilisé comme vecteur ou diluant peut être utilisé dans les compositions de la présente invention, qu'il s'agisse, par exemple, des gommes, des amidons (par exemple amidon de maïs, amidon prégélatinisé), des sucres (par exemple lactose, mannitol, saccharose, dextrose), des matériaux cellulosiques (par exemple cellulose microcristalline), des acrylates (par exemple polyméthylacrylate), du carbonate de calcium, de l'oxyde de magnésium, du talc, ou des mélanges de ceux-ci. Un diluant préféré est le monohydrate de lactose. Lorsqu'elles se présentent sous la forme de comprimés solides, les compositions peuvent comprendre, en plus, un agent désintégrant (par exemple cellulose microcristalline), un lubrifiant (par exemple béhénate de glycérol) et un agent de glissement (par exemple dioxyde de silice colloïdale).

Dans un mode de réalisation préféré, la composition pharmaceutique comprend du chlorhydrate d'idazoxan, du monohydrate de lactose, de la cellulose microcristalline, du béhénate de glycérol et du dioxyde de silice colloïdale. De manière plus préférée, la composition pharmaceutique comprend 10% en poids de chlorhydrate d'idazoxan, 61,46% en poids de monohydrate de lactose, 26,34% en poids de cellulose microcristalline, 2% en poids de béhénate de glycérol et 0,2% en poids de dioxyde de silice colloïdale.

Dans un mode de réalisation de la présente invention, la composition est formulée sous forme de comprimés qui possèdent de préférence une masse comprise entre 50 mg et 1000 mg, de préférence entre 50 et 600 mg, de manière plus préférée environ 100 mg, 200 mg, 300 mg, 400 mg ou environ 500 mg.

La présente invention englobe également des compositions pharmaceutiques comprenant une quelconque forme physique solide ou liquide de l'un quelconque des polymorphes de l'idazoxan décrits ici. Par exemple, les polymorphes de l'idazoxan peuvent se présenter sous une forme cristalline et avoir une taille de particule quelconque. Les particules des polymorphes de l'idazoxan peuvent être micronisées ou agglomérées, peuvent se présenter sous la forme de granules particulaires, de poudres ou de toute autre forme physique solide; le mode de réalisation préféré correspond à une forme solide.

Il est particulièrement avantageux de formuler les compositions orales sous forme posologique unitaire pour une administration plus facile et une posologie uniforme. La forme posologique unitaire comme utilisée ici renvoie à des unités physiquement discrètes, adaptées à une posologie unitaire pour le sujet à traiter; chaque unité comprenant une quantité prédéterminée de composé actif calculée pour produire l'effet thérapeutique désiré en association avec le vecteur pharmaceutique requis. Les spécifications des formes posologiques unitaires de l'invention sont dictées par les caractéristiques uniques du composé actif et par l'effet thérapeutique spécifique devant être obtenu, ainsi que par les limitations inhérentes à l'art de la composition d'un tel composé actif pour le traitement des individus, ces spécifications dépendant également directement des éléments précédents.

Les compositions pharmaceutiques peuvent être contenues dans un conditionnement, un emballage ou un appareil distributeur en même temps que la notice d'emploi.

Avantageusement, lesdits comprimés se présentent dans un conditionnement étanche, qui peut être constitué par un pilulier en polypropylène ou en polyéthylène haute densité, un sachet en aluminium ou un blister "tout aluminium".

Les compositions pharmaceutiques selon la présente invention peuvent aussi être présentées sous forme adaptée à l'administration parentérale, intrapéritonéale, intraveineuse, intra-artérielle, transdermale, sublinguale, intramusculaire, rectale, transbuccale, intranasale, liposomale, vaginale ou intra-occulaire ou sous une forme adaptée à la délivrance locale par un cathéter ou un stent.

Un aspect supplémentaire de la présente invention vise un procédé de fabrication desdites compositions par compression directe d'un mélange de poudre. Ce procédé de fabrication est préféré à un procédé faisant intervenir une étape de granulation avec un liquide de mouillage car de façon surprenante un liant classique comme la povidone est incompatible avec ce principe actif. Alternativement, le procédé par compression directe comprend une étape de granulation sèche, par exemple par compactage, qui précède l'étape de compression. Dans le procédé de fabrication par compression, le sel d'idazoxan à une granulométrie exprimée par son diamètre moyen compris entre 50 et 250 microns, de préférence compris entre 75 et 150 microns et plus particulièrement voisin de 100 à 125 microns. Par ailleurs, le sel d'idazoxan ou l'hydrate d'idazoxan a une densité vrac comprise entre 0,4 et 0,8 et préférentiellement comprise entre 0,5 et 0,7 et de manière encore plus préférée proche de 0,6.

La présente invention vise également l'utilisation desdites compositions pour la fabrication d'un médicament, notamment pour la fabrication d'un médicament destiné à la prévention et/ou au traitement des troubles du système nerveux central. De préférence, le médicament sous la forme d'un comprimé est destiné au traitement de la dépression, de la maladie de Parkinson, et des désordres psychotiques sévères, tels la schizophrénie et les maladies schizo-affectives.

Selon un autre mode préféré de réalisation, le médicament est utilisé en association avec un second médicament, de préférence un neuroleptique atypique, encore plus avantageusement un neuroleptique atypique possédant une plus grande affinité antagoniste pour le récepteur D₂ que pour le récepteur alpha-2-noradrénergique, pour le traitement des maladies mentales psychotiques sévères, notamment la schizophrénie et les maladies schizo-affectives. Pour une définition des neuroleptiques atypiques, voir la demande de brevet américain US 2004/0127489 et la demande internationale WO 2004/011031 basées sur les demandes US 60/398, 718 et US 60/398,719 déposées le 29 juin 2002. Parmi les neuroleptiques atypiques, il convient de citer l'olanzapine, la quétiapine, la rispéridone, la sertindole, la ziprasidone. De manière plus préférée, il s'agit de l'olanzapine. L'administration du dit premier médicament et du dit second médicament est réalisée de manière simultanée, séparée ou étalée dans le temps, dans n'importe quel ordre.

D'autres caractéristiques et avantages de l'invention apparaissent dans la suite de la description avec les exemples représentés ci-après. Dans ces exemples on se référera aux figures suivantes. Ces figures et exemples sont destinés à illustrer la présente invention.
**FIGURE 1:** Diffractogramme des rayons X pour le polymorphe du chlorhydrate d'idazoxan de forme I.
**FIGURE 2**: Diffractogramme des rayons X pour le polymorphe du chlorhydrate d'idazoxan de forme II.
**FIGURE 3:** Diffractogramme des rayons X pour le polymorphe du chlorhydrate d'idazoxan de forme III.
**FIGURE 4:** Diffractogramme des rayons X pour le polymorphe du chlorhydrate d'idazoxan de forme IV.
**FIGURE 5:** Diffractogramme des rayons X pour le monohydrate du chlorhydrate d'idazoxan de forme V.
**FIGURE 6:** Diffractogramme des rayons X pour le monohydrate de chlorhydrate d'idazoxan de forme VI.
**FIGURE 7:** Thermogramme d'analyse thermique différentielle pour le polymorphe du chlorhydrate d'idazoxan de forme I, II, III, V.
**FIGURE 8:** Thermogramme d'analyse thermique différentielle pour le polymorphe du chlorhydrate d'idazoxan de forme IV.

### Exemple 1: Etude de stabilité des formulations contenant du chlorhydrate d'idazoxan.

Le chlorhydrate d'idazoxan appartient à la classe des alpha-2-antagonistes. Le nom chimique de cette molécule est 2-(2 imidazolinyl)-1,4 benzodioxan hydrochloride, et sa formule développée est: C₁₁H₁₂N₂O₂, HCl.

L'idazoxan se présente sous la forme d'une poudre blanche à sensiblement blanche, légèrement hygroscopique, facilement soluble dans l'eau et le méthanol, soluble à assez soluble dans l'éthanol. Du fait de sa légère hygroscopicité, elle doit être conservée en conditionnement étanche, à l'abri de l'humidité. Durant tous les essais effectués, cette molécule présentait les caractéristiques suivantes:
■ Teneur en chlorhydrate d'idazoxan comprise entre 99,0 et 101,0%
■ Teneur individuelle en impuretés connues ≤ 0,2%
■ Teneur individuelle en impuretés inconnues ≤ 0,1%
■ Teneur totale en impuretés ≤ 1,0%.
L'impureté principale du chlorhydrate d'idazoxan est appelée 791011: 1,4-benzodioxane-2[(N-éthyl-2-amino)-carboxamide].
La stabilité du principe actif a été étudiée dans différentes conditions de stress:
■ + 4°C dans un flacon fermé (référence)
■ 50°C, flacon ouvert
■ 40°C, 30% HR flacon ouvert
■ 40°C, 75% HR flacon ouvert.

Quelles que soient les conditions, le chlorhydrate d'idazoxan ne subit aucun changement organoleptique et aucune instabilité chimique notable n'est observée, même pour des humidités relatives élevées (pourcentage en 791011 ≤ 0,03% et absence d'impuretés inconnues).

Des études de compatibilité ont été réalisées à 40°C, 75% HR pendant 1 mois entre le chlorhydrate d'idazoxan et divers excipients classiques des formes sèches.

Les résultats obtenus sont exposés dans le tableau 1 ci-dessous et expriment la différence surprenante de comportement des mélanges binaires entre le contrôle juste après fabrication et le contrôle après 1 mois en condition stressante alors que le principe actif seul est parfaitement stable.

**Tableau 1**

| **Excipient** | **Ratio Principe actif/ Excipient** | **% 791011** | **Fonction** |
|---|---|---|---|
| Lactose monohydraté | 1/4 | 0,1 | Diluants |
| Lactose anhydre | 1/4 | 0,3 | |
| Mannitol | 1/4 | 0,3 | |
| Sorbitol | 1/4 | 40,8 | |
| Cellulose microcristalline | 1/2 | 3,7 | Diluant/Liant/ désintégrant |
| Carboxyméthylcellulose calcique | 1/2 | 4,4 | Désintégrant |
| Povidone | 4/1 | 8,5 | Liant |
| Hydropropylméthyl-cellulose (HPMC) | 4/1 | 1,5 | Liant |
| Stéarate de magnésium | 4/1 | 17,0 | Lubrifiant |
| Talc | 4/1 | 1,0 | Antiadhérent |

Les excipients les plus compatibles avec le chlorhydrate d'idazoxan sont le lactose, le mannitol, la cellulose microcristalline et l'HPMC. Cette étude montre la grande réactivité du chlorhydrate d'idazoxan en présence d'excipients, classiquement utilisés en formulation pharmaceutique.

Pour réaliser les premières formules de cette molécule, une forme gélule classique à base de gélatine a été développée.

Le lactose ou le mannitol ont été utilisés comme diluants en raison des résultats des compatibilités binaires. Ces diluants ont été associés à des excipients classiques de lubrification: talc et stéarate de magnésium.

Ces formules ont été lubrifiées par du talc et du stéarate de magnésium malgré le résultat négatif obtenu avec le stéarate de magnésium, ceci afin de confirmer cette incompatibilité au taux réel d'utilisation du stéarate de magnésium.

Les gélules fabriquées ont été conditionnées en conditionnement étanche (pilulier polypropylène) en raison de la légère hygroscopicité du principe actif et du risque d'ouverture de cycle avec la formation de l'impureté 791011 (produit lui-même réactif).

La composition des formules réalisées est présentée dans le tableau 2 ci-dessous:

**Tableau 2**

| **Composant** | **Formule 1** | **Formule 2** | **Formule 3** | **Fonction** |
|---|---|---|---|---|
| Chlorhydrate d'idazoxan | 20 mg | 20 mg | 20 mg | Principe actif |
| Lactose anhydre | 130,2 mg | 0 | 0 | Diluants |
| Lactose monohydraté | 0 | 0 | 158 mg | |
| Mannitol | 0 | 130,2 mg | | |
| Cellulose microcristalline | 0 | 0 | 20 mg | |
| Stéarate de magnésium | 0,8 mg | 0,8 mg | 2 mg | Lubrifiant |
| Talc | 4,0 mg | 4,0 mg | 0 | |
| Gélules taille 3 | | | | |

Après 1 mois de stabilité en pilulier à 40 °C, 75 % HR, les analyses montrent un taux de produit de dégradation rédhibitoire (cf. Tableau 3 ci-dessous):

**Tableau 3**

| | **Formule 1** | **Formule 2** | **Formule 3** |
|---|---|---|---|
| % de 791011 | 3,0 % | 2,0 % | 2,9 % |

Il n'est donc pas possible de formuler le chlorhydrate d'idazoxan dans une formulation simple en gélules; a posteriori, il a été démontré que ce principe actif est incompatible avec la gélatine. De plus, les formules démontrent l'incompatibilité du stéarate de magnésium.

La formulation en comprimé s'avère nécessaire afin d'assurer une stabilité satisfaisante du principe actif.

Par ailleurs, une alternative au stéarate de magnésium a été identifiée avec le béhénate de glycérol.

Afin d'assurer une bonne homogénéité de répartition du principe actif et ceci le plus indépendamment possible des caractéristiques physiques du principe actif et des excipients, la fabrication de comprimés par granulation humide est étudiée dans un premier temps.

La granulation humide a été effectuée dans un mélangeur granulateur haute vitesse avec de l'eau. Les granulés obtenus sont séchés en lit d'air fluidisé puis calibrés sur une grille de 0,4 mm.

Deux modes opératoires ont été retenus:
■ Soit granulation de la totalité du diluant, du liant/délitant et du principe actif. Après granulation, séchage et tamisage, la lubrification est effectuée par mélange du grain avec le lubrifiant.
■ Soit granulation de la moitié du diluant, de la moitié du liant/délitant avec la totalité du principe actif. Après granulation, séchage et tamisage, mélange du grain avec l'autre moitié du liant/désintégrant et du diluant puis ajout du lubrifiant en dernier et mélange.

Les comprimés ont ensuite été conditionnés en blisters tout aluminium ou en piluliers étanches.

Les formules (en mg) mises en oeuvre et les résultats pharmacotechniques sont respectivement décrits dans les tableaux 4 et 5 ci-dessous:

**Tableau 4**

| **Matière première** | **Formule 4** | **Formule 5** | **Formule 6** | **Formule 7** |
|---|---|---|---|---|
| Chlorhydrate d'idazoxan | 20(5%) | 20(10%) | 30(10%) | 30(20%) |
| Diluant: lactose | 332 | 156 | 205 | 104 |
| Lubrifiant: béhénate de glycérol | 8 | 4 | 5 | 4 |
| Liant/désintégrant: cellulose microcristalline | 40.(10%) | 20(10%) | 60(20%) | 21(15%) |
| TOTAL | 400g | 200 | 300 | 140 |

La granulation a été effectuée avec 10% de liquide de mouillage par rapport à la masse totale du comprimé.

**Tableau 5**

| **Matière première** | **Formula 4** | **Formule 5** | **Formule 6** | **Formule 7** |
|---|---|---|---|---|
| % 791011 | 4,54 % | 0,44 % | 5,2 % | < 0,2 % |
| Dureté | 30 N | 40 N | 40 N | Nulle |
| Clivage | - | - | - | +++ |

Des études complémentaires menées avec une formule qualitativement équivalente à la formule 5 ont montré qu'une teneur de 10% de chlorhydrate d'idazoxan par rapport à la masse totale du comprimé, associée à une teneur de 10% de cellulose microcristalline, était celle qui permettait d'obtenir le meilleur compromis entre la dureté des comprimés et le clivage lors de la compression.

Les résultats obtenus en faisabilité (dureté et clivage) sont satisfaisants dans la fourchette de 5 à 10% de chlorhydrate d'idazoxan, associés à 8 à 15 % de liant/désintégrant, type cellulose microcristalline.

Ainsi, des expériences ont été poursuivies jusqu'à l'optimisation de la formulation N°5; dans ce but deux quantités de liquide de mouillage ont été testées: 5 et 20%.
■ Formule 8: 5% de liquide de mouillage
■ Formule 12: 20% de liquide de mouillage

Les résultats concernant ces formules sont exprimés dans le tableau 6 ci-dessous. Le taux d'impureté a été déterminé après un mois à 40°C, 75% HR.

**Tableau 6**

| **% de liquide de mouillage** | **Taux d'impureté 791011** | **Dureté** | **Clivage** | **CV de teneur** |
|---|---|---|---|---|
| 5% (formule 5bis) | 1,5 % | 20 N | +++ | 8 % |
| 20% (formule 5ter) | 2,3 % | 20 N | - | 7 % |

Le taux de dilution étudié de principe actif (10%) ne permet pas d'atteindre des homogénéités de mélange satisfaisantes (coefficient de variation de teneur >6%), ceci de 5 à 20% d'eau utilisée en mouillage lors de la granulation.

De plus, les taux de dégradation obtenus restent, quoique légèrement inférieurs, comparables à ceux obtenus avec les formules testées en gélule (compris entre 1,5 et 2,3% en produit 791011).

Au niveau de la fabrication de comprimés, le procédé alternatif à la granulation humide est la compression directe. Compte tenu des résultats obtenus en granulation il est donc logique d'étudier les possibilités de ce procédé concernant le chlorhydrate d'idazoxan.

Les caractéristiques pharmacotechniques du principe actif sont les suivantes:
■ Masse volumique vrac comprise entre 0,5 et 0,8 g/ml
■ Diamètre moyen compris entre 100 et 300 µm
■ Aptitude favorable à la compression: sur une machine à comprimer alternative, si une force de 10 000 Newtons est appliquée, la dureté des comprimés obtenus avec du principe actif pur (Volume = 1 cm³) est comprise entre 10 et 30 N sur un format rond d'1 cm² de surface.

Les formules mises en oeuvre sont présentées dans le tableau 7 ci-dessous:

**Tableau 7**

| **Matières premières** | **Formule 8 (mg)** | **Formule 9 (mg)** | **Formule 10 (mg)** | **Formule 11 (mg)** | **Fonction** |
|---|---|---|---|---|---|
| Chlorhydrate d'idazoxan | 20 | 20 | 20 | 20 | Principe actif |
| Lactose monohydraté | 158 | 152 | 0 | 122,92 | Diluant |
| Mannitol | 0 | 0 | 158 | 0 | |
| Cellulose micro-cristalline | 20 | 20 | 20 | 52,68 | Liant/ désintégrant |
| Bénéhate de glycérol | 0 | 8 | 0 | 4 | Lubrifiant |
| Stéarate de magnésium | 2 | 0 | 2 | 0 | Lubrifiant |
| Silice colloïdale anhydre | 0 | 0 | 0 | 0,4 | Agent d'écoulement |
| TOTAL | 200 mg | | | | |

Les comprimés obtenus ont été conditionnés en blister étanche (tout aluminium).

Le tableau 8 ci-dessous décrit les résultats pharmacotechniques obtenus après fabrication, ainsi que le pourcentage d'impureté retrouvé après 1 mois à 40°C, 75 HR.

**Tableau 8**

| **Résultats** | **Formule 8** | **Formule 9** | **Formule 10** | **Formule 11** |
|---|---|---|---|---|
| Dureté (format D8R10) | 20 Newtons | 21 Newtons | 25 Newtons | 50 Newtons |
| Clivage | ++ | ++ | +++ | + |
| Collage | + | ++ | +++ | + |
| C.V. de teneur | 5,1 % | 8 % | 7,4 % | 4,1 % |
| % impureté 791011 | 1,78 % | 2,41 % | 1,12 % | 1,03 % |

La formule 11 a la meilleure dureté, présente le plus faible taux d'impureté et a donc été retenue. Les formules 8, 9, 10 ont des duretés trop faibles pour pouvoir envisager une fabrication industrielle sur machine rotative. De plus, le clivage et le collage constatés sont rédhibitoires. Cette formule 11 obtenue par compression directe est donc le meilleur compromis pour atteindre une faisabilité technique de la forme en compression directe et une stabilité satisfaisante du principe actif.

La stabilité est également satisfaisante par un conditionnement étanche type blister aluminium 45 µm operculé par un aluminium de 20 µm (voir tableau 9 ci-après).

**Tableau 9**

| Stabilité 24 mois Formule 11 | | | | | | |
|---|---|---|---|---|---|---|
| Condition: 25°C 60% Humidité Relative | | | | | | |
| | 0 mois | 1 mois | 3 mois | 6 mois | 12 mois | 24 mois |
| Teneur en chlorhydrate d'idazoxan (mg/comprimé) | 19,51 | 19,66 | 20,00 | 19,88 | 18,84 | 19,16 |
| % de produit de dégradation 791011 | 0,24 | 0,66 | 0,74 | 1,06 | 1,19 | 1,64 |

### Exemple 2: Exemples de formulations

| Formulation 1: | |
|---|---|
| Chlorhydrate d'idazoxan | 5% |
| Cellulose microcristalline | 10% |
| Béhénate de glycérol | 5% |
| Silice colloïdale | 0,1% |
| Lactose monohydraté | QSP 100% |

Cette formule permet de fabriquer des comprimés titrant entre 10 et 30 mg de chlorhydrate d'idazoxan conditionnés en blisters tout aluminium.

| Formulation 2: | |
|---|---|
| Chlorhydrate d'idazoxan | 5% |
| Cellulose microcristalline | 40% |
| Béhénate de glycérol | 1% |
| Silice colloïdale | 0,1% |
| Lactose monohydraté | QSP 100% |

Cette formule permet de fabriquer des comprimés titrant entre 10 et 30 mg de chlorhydrate d'idazoxan conditionnés en blisters tout aluminium.

| Formulation 3: | |
|---|---|
| Chlorhydrate d'idazoxan | 5% |
| Cellulose microcristalline | 40% |
| Béhénate de glycérol | 5% |
| Silice colloïdale | 0,5% |
| Lactose monohydraté | QSP 100% |

Cette formule permet de fabriquer des comprimés titrant entre 10 mg et 30 mg de chlorhydrate d'idazoxan conditionnés en blisters tout aluminium.

| Formulation 4: | |
|---|---|
| Chlorhydrate d'idazoxan | 20% |
| Cellulose microcristalline | 10% |
| Béhénate de glycérol | 5% |
| Silice colloïdale | 0,1% |
| Lactose monohydraté | QSP 100% |

Cette formule permet de fabriquer des comprimés titrant entre 10 et 30 mg de chlorhydrate d'idazoxan conditionnés en blisters tout aluminium.

| Formulation 5: | |
|---|---|
| Chlorhydrate d'idazoxan | 20% |
| Cellulose microcristalline | 40% |
| Béhénate de glycérol | 2% |
| Silice colloïdale | 0,1% |
| Lactose monohydraté | QSP 100% |

Cette formule permet de fabriquer des comprimés titrant entre 10 et 30 mg de chlorhydrate d'idazoxan conditionnés en blisters tout aluminium.

| Formulation 6: | |
|---|---|
| Chlorhydrate d'idazoxan | 20% |
| Cellulose microcristalline | 40% |
| Béhénate de glycérol | 5% |
| Silice colloïdale | 0,1% |
| Lactose monohydraté | QSP 100% |

Cette formule permet de fabriquer des comprimés titrant entre 10 et 30 mg de chlorhydrate d'idazoxan conditionnés en blisters tout aluminium.

| Formulation 7: | |
|---|---|
| Chlorhydrate d'idazoxan | 10% |
| Cellulose microcristalline | 26,34% |
| Béhénate de glycérol | 2% |
| Silice colloïdale | 0,2% |
| Lactose monohydraté | QSP 100% |

Cette formule permet de fabriquer des comprimés titrant entre 10 et 30 mg de chlorhydrate d'idazoxan conditionnés en blisters tout aluminium.

NB: Les résultats des analyses physico-chimiques dé ces comprimés exemplifiés (formulations 1 à 7), fabriqués sur machine rotative industrielle, après mise en conditionnement étanche, ont montré:
■ un temps de désagrégation des comprimés inférieur à 15 minutes juste après fabrication et au cours du temps,
■ un pourcentage de principe actif dissous supérieur à 80% en 30 minutes après fabrication et au cours du temps,
■ des C.V. de teneur inférieure à 6%,
■ une teneur moyenne des comprimés à ± 5% du dosage théorique en principe actif,
■ une bonne stabilité au cours du temps, la somme des impuretés étant inférieure à 2% après 24 mois à 25°C, 60 HR,
■ une bonne qualité microbiologique après fabrication et au cours du temps.

### Exemple 3: Profil de dégradation et stabilité

Une étude de photostabilité selon les critères ICH n'a montré aucune dégradation. Par ailleurs, une étude de stabilité en condition long terme et accélérée a été réalisée, et le tableau 10 montre la stabilité à long terme du polymorphe de forme I:

**Tableau 10: stabilité à long terme (en mois)**

| **Lot de la forme I** | **25°C/60%HR¹** | **30°C/70%HR** | **40°C/75%HR** |
|---|---|---|---|
| OP2 | 24 | 24 | 9 |
| 500 | 24 | 24 | 6 |
| 501 | 24 | 24 | 6 |
| 503 | 9 | 6 | 6 |
| 504 | 9 | 6 | 6 |

| | | | |
|---|---|---|---|
| ¹HR = humidité relative | | | |

### Exemple 4: Analyse de la solubilité du polymorphe de forme I

Le tableau 11 montre la solubilité du polymorphe de forme I dans divers solvants.

**Tableau 11**

| **Lot de la forme I** | **Eau** | **Ethanol** | **Acétate d'éthyle** |
|---|---|---|---|
| OP2 | Librement soluble | Soluble | Pratiquement insoluble |
| 500 | Librement soluble | Soluble | Pratiquement insoluble |
| 501 | Librement soluble | Soluble | Pratiquement insoluble |
| 503 | Librement soluble | Soluble | Pratiquement insoluble |
| 504 | Librement soluble | Soluble | Pratiquement insoluble |
| 507 | Librement soluble | Modérément soluble | Pratiquement insoluble |
| 508 | Librement soluble | Soluble | Pratiquement insoluble |

### Exemple 5: Procédés de synthèse

### 5.1. Procédés de synthèse du chlorhydrate d'idazoxan de l'art antérieur

Le chlorhydrate d'idazoxan peut être synthétisé selon la méthode publiée décrite ci-dessous:

*Préparation* de *chlorhydrate* de *2-[2-(1,4-benzodioxanyl)]-2-imidazoline.* Une solution de méthoxyde de sodium (1,45 g) dans du méthanol (20 ml) est ajoutée en l'espace d'une minute à une solution agitée de 2-cyano-1,4-benzodioxane (145 g) dans du méthanol (870 ml) à température ambiante. Après agitation pendant encore 4 heures à température ambiante, la solution est refroidie et de l'éthylènediamine (64,7 g) est ajoutée goutte à goutte à la température de 5°C. Une solution de chlorure d'hydrogène dans du méthanol (134 g de solution contenant 34,8 g de chlorure d'hydrogène) est alors ajoutée à la solution agitée en l'espace de 2 heures et à la température de 5°C. Après encore 20 heures à 0-10°C, le dihydrochlorure d'éthylènediamine précipité est éliminé par filtration et le filtrat est réduit à 300 g sous vide à 40°C. Du dihydrochlorure d'éthylènediamine est encore éliminé et le filtrat restant est soumis à évaporation sous vide à 40°C jusqu'à séchage complet. Le résidu solide (225 g) est agité avec du dichlorométhane (1,1 litres) et le chlorure d'hydrogène sec est mis à barboter à 5-10°C jusqu'à obtention d'un léger excès. Le produit brut est ensuite éliminé par filtration (172 g) et combiné avec une seconde récolte (24 g) obtenue par concentration du filtrat sous vide à 40°C. La cristallisation de ces deux récoltes à partir de l'éthanol avec filtration à chaud et concentration du filtrat sous vide jusqu'à obtention de 384 g donne un produit cristallin blanc cassé (175,5 g, 81 %), point de fusion 207-208°C. Si l'éthylènediamine et le chlorure d'hydrogène sont ajoutés dans l'ordre inverse dans le méthanol, on obtient un rendement similaire.

### 5.2. Procédés de synthèse du chlorhydrate d'idazoxan avec polymorphes

Le chlorhydrate d'idazoxan comprenant un polymorphe de l'une quelconque des formes I peut être synthétisé selon la méthode décrite ci-dessous:
(a) *Etape 1*. La première étape consiste en l'obtention de 2-cyano-1,4-benzodioxane à partir de la réaction du catéchol et du 2-chloroacrylonitrile en présence de carbonate de potassium pulvérisé et d'un catalyseur, le bromure de tétrabutylammonium, dans un mélange de toluène et de dioxane. La réaction est réalisée à une température comprise entre 80 et 90°C . Après une double décoloration, le toluène s'évapore et on obtient un extrait huileux de 2-cyano-1,4-benzodioxane sous forme racémique.
(b) *Etape 2*. En présence de méthoxyde de sodium, le 2-cyano-1,4-benzodioxane donne un imidate non isolé, qui est encore cyclisé en idazoxan en présence d'éthylènediamine et d'acide chlorhydrique. L'excès d'éthylènediamine est cristallisé par addition d'acide chlorhydrique, puis éliminé par filtration. La recristallisation du chlorhydrate d'idazoxan est réalisée dans de l'éthanol. La substance médicamenteuse obtenue correspond à la forme I racémique.

Une étape de purification supplémentaire à l'aide de différents solvants permet d'obtenir différents polymorphes du chlorhydrate d'idazoxan.
(c) *Etape 3* - Purification. La purification du composé final est possible quand l'un quelconque de ses attributs ne respecte pas les spécifications.

Pour les impuretés telles que la cendre sulfatée, les métaux lourds et le contenu en éthylènediamine, le retraitement consiste en un retour à la base suivi d'un lavage liquide-liquide avant le retour à la forme hydrochlorure. Une recristallisation est alors réalisée.

En cas de non-conformité en relation avec la clarté de la solution, la coloration de la solution, le pH de la solution, le contenu en 2-chloroacrylonitrile et les substances associées (chromatographie liquide haute pression), le retraitement consiste en une recristallisation.

En cas de non-conformité en relation avec le contenu en eau et les solvants résiduels, le séchage est poursuivi.

Cette étape de purification/recristallisation peut être réalisée au moyen des solvants suivants: acétonitrile, n-propanol, méthanol, éthanol, butanol-1 et n-butanol. La forme III est obtenue par recristallisation de la forme I avec de l'éthanol.

La forme IV est obtenue par recristallisation de la forme I avec du butanol-1.

Un monohydrate de chlorhydrate d'idazoxan (forme V) est obtenu par réempâtage de la forme I dans 5 volumes d'un mélange acétone-eau.

La forme VI peut être obtenue en maintenant une suspension d'une partie en poids sec de forme I dans 7 volumes d'éthanol à 100° sous constante agitation et à température ambiante pendant 1 à 4 jours, filtrant sous vide et en séchant dans un four sous vide à 70°C pendant une nuit.

La forme VI peut également être obtenue en maintenant une solution de forme I dans l'éthanol sous agitation constante et à haute température, en distillant l'éthanol sous vide jusqu'à évaporation partielle du solvant de manière à induire la cristallisation, en refroidissant ensuite la solution à 0° sur une période de 3 heures, et en filtrant et en séchant dans un four sous vide à 70°C pendant la nuit.

### Exemple 6: Analyse cristallographique par diffraction des rayons X sur poudre des polymorphes I à VI de l'idazoxan

Une cristallographie par diffraction des rayons X sur poudre a été réalisée sur les formes I à VI de l'idazoxan obtenues conformément au procédé de la présente invention comme détaillé dans le tableau 12 ci-dessous.

**Tableau 12:**

| *Echantillons de po lymorphes de l'idazoxan analysés par cristallographie par diffraction des* rayons X *sur poudre* | |
|---|---|
| Echantillon de polymorphes | Procédé |
| Forme I | Exemples 5.1 et 5.2 |
| Forme II | Exemple 5.1 |
| Forme III | Exemple 5.2 (éthanol) |
| Forme IV | Exemple 5.2 (butanol-1) |
| Forme V | Exemple 5.2 (eau acétone) |
| Forme VI | Exemple 5.2 |

Les échantillons ont été analysés au moyen d'un appareil Philips PW 1730 équipé d'un goniomètre horizontal CGR de type C et d'une anticathode Cu (A=1,54051 A), fonctionnant à 40 kV et 20 mA.

La préparation de l'échantillon a été réalisée selon la procédure standard d'exploitation, conformément aux instructions du fabricant, en utilisant une plaque porte-échantillons sans parasites. Les échantillons ont été analysés suite à un léger broyage au mortier et au pilon pour garantir une bonne homogénéité.

Il a été observé que la fusion et la décomposition du chlorhydrate d'idazoxan ne permettent pas une étude thermo-analytique des polymorphes dans cette plage de température. Seule la cristallographie par diffraction des rayons X peut permettre la découverte de différentes formes cristallines.

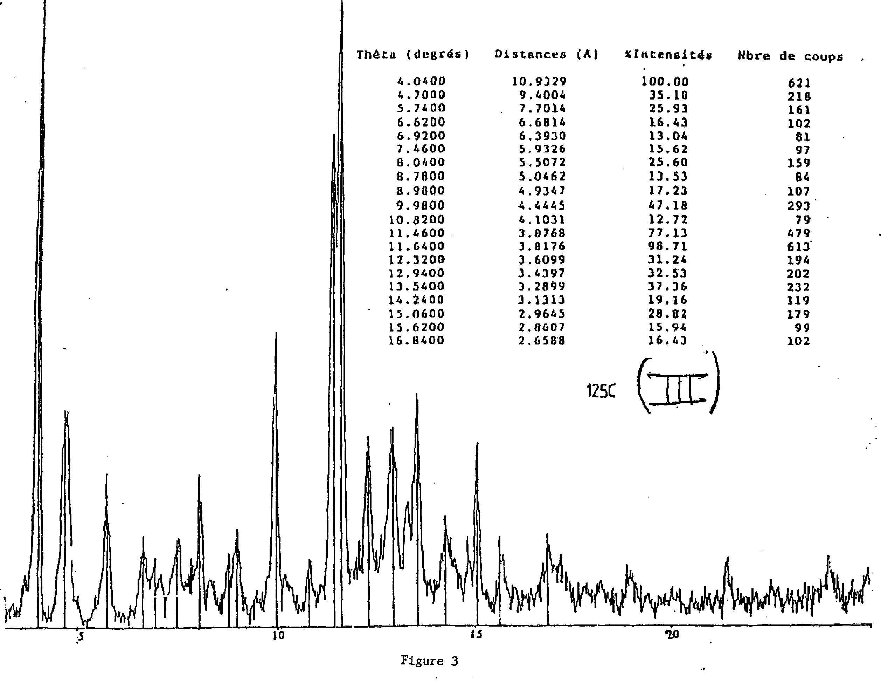

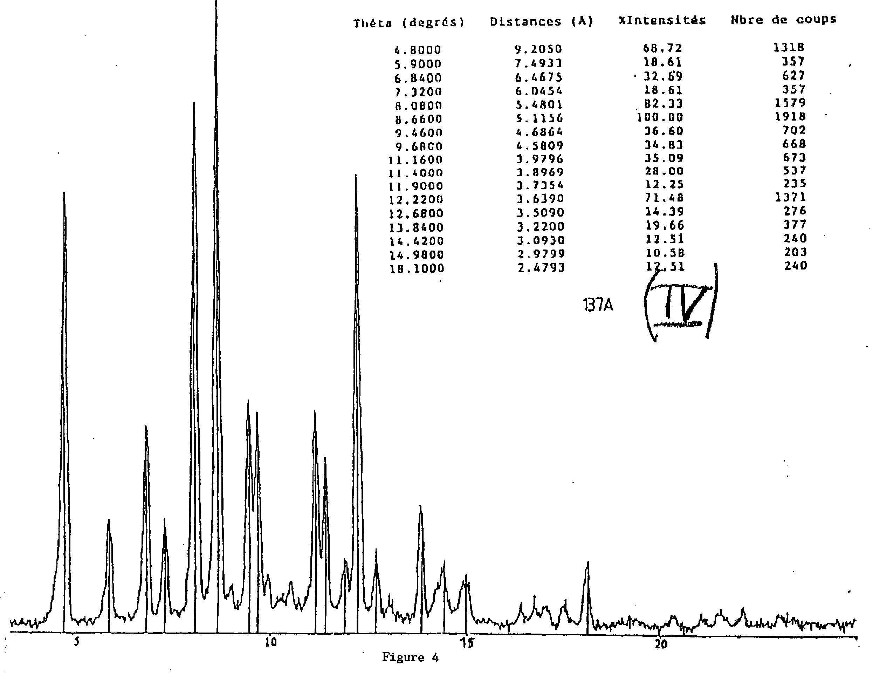

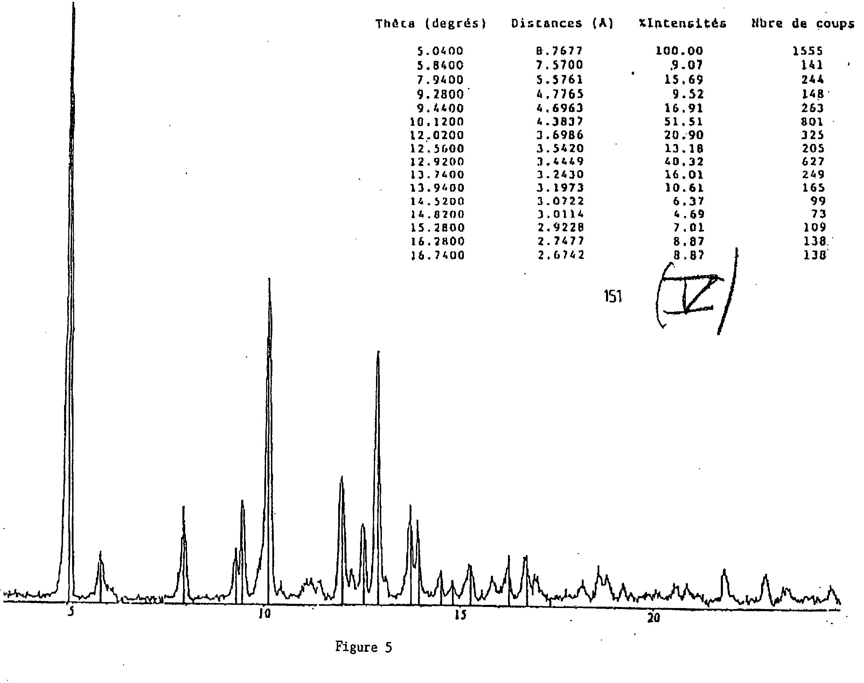

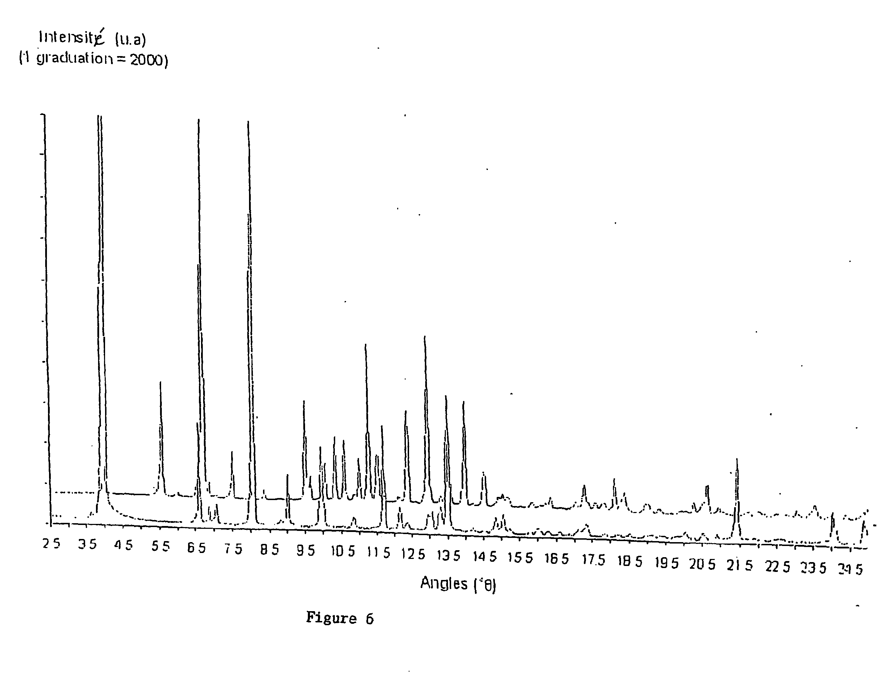

La figure 1 représente le diffractogramme des rayons X pour le polymorphe de l'idazoxan de forme I, fabriqué conformément au procédé de la présente invention (exemple 5.2); la figure 2 représente le diffractogramme des rayons X pour le polymorphe de l'idazoxan de forme II, fabriqué selon l'art antérieur (exemple 5.1); les figures 3 et 4 représentent le diffractogramme des rayons X pour les polymorphes de l'idazoxan de forme III et IV, respectivement (exemple 5.2); la figure 5 représente le diffractogramme des rayons X pour le polymorphe de l'idazoxan de forme V (exemple 5.2); la figure 6 représente le diffractogramme des rayons X pour le polymorphe de l'idazoxan de forme VI. Les données correspondant aux diffractogrammes des rayons X sont présentées dans les tableaux 13 à 18 ci-dessous:

**Tableau 13: Polymorphe de l'idazoxan de forme I**

| **Pic** | **Thêta (degré)** | **D (Å)** | **Pic** | **Thêta (degré)** | **D (Å)** |
|---|---|---|---|---|---|
| 1 | 4,0200 | 10,9872 | 10 | 12,1400 | 3,6626 |
| 2 | 6,6400 | 6,6613 | 11 | 12,3800 | 3,5927 |
| 3 | 6,9000 | 6,4115 | 12 | 12,9800 | 3,4293 |
| 4 | 7,0800 | 6,2493 | 13 | 13,3000 | 3,3482 |
| 5 | 8,0800 | 5,4801 | 14 | 13,5200 | 3,2947 |
| 6 | 9,0000 | 4,9238 | 15 | 14,9000 | 2,9956 |
| 7 | 9,9600 | 4,4534 | 16 | 15,0600 | 2,9645 |
| 8 | 10,8400 | 4,0956 | 17 | 15,2400 | 2,9303 |
| 9 | 11,7200 | 3,7919 | 18 | 21,4000 | 2,1110 |

**Tableau 14: Polymorphe de l'idazoxan de forme II**

| **Pic** | **Thêta (degré)** | **D (Å)** | **Pic** | **Thêta D (Å) (degré)** | |
|---|---|---|---|---|---|
| 1 | 4,7400 | 9,3213 | 8 | 12,3000 | 3,6157 |
| 2 | 5,7200 | 7,7283 | 9 | 12,9400 | 3,4397 |
| 3 | 6,6800 | 6,6216 | 10 | 13,5400 | 3,2899 |
| 4 | 7,5000 | 5,9012 | 11 | 14,3000 | 3,1185 |
| 5 | 8,9200 | 4,9676 | 12 | 15,6800 | 2,8500 |
| 6 | 9,9600 | 4,4534 | 13 | 16,8600 | 2,6557 |
| 7 | 11,5200 | 3,8569 | 14 | 18,9000 | 2,3779 |

**Tableau 15: Polymorphe de l'idazoxan de forme III**

| **Pic** | **Thêta (degré)** | **D (Å)** | **Pic** | **Thêta (degré)** | **D (Å)** |
|---|---|---|---|---|---|
| 1 | 4,0400 | 10,9329 | 11 | 10,8200 | 4,1031 |
| 2 | 4,7000 | 9,4004 | 12 | 11,4600 | 3,8768 |
| 3 | 5,7400 | 7,7014 | 13 | 11,6400 | 3,8176 |
| 4 | 6,6200 | 6,6814 | 14 | 12,3200 | 3,6099 |
| 5 | 6,9200 | 6,3930 | 15 | 12,9400 | 3,4397 |
| 6 | 7,4600 | 5,9326 | 16 | 13,5400 | 3,2899 |
| 7 | 8,0400 | 5,5072 | 17 | 14,2400 | 3,1313 |
| 8 | 8,7800 | 5,0462 | 18 | 15,0600 | 2,9645 |
| 9 | 8,9800 | 4,9347 | 19 | 15,6200 | 2,8607 |
| 10 | 9,9800 | 4,4445 | 20 | 16,8400 | 2,6588 |

**Tableau 16: Polymorphe de l'idazoxan de forme IV**

| **Pic** | **Thêta (degré)** | **D (Å)** | **Pic** | **Thêta (degré)** | **D (Å)** |
|---|---|---|---|---|---|
| 1 | 4,8000 | 9,2050 | 10 | 11,4000 | 3,8969 |
| 2 | 5,9000 | 7,4933 | 11 | 11,9000 | 3,7354 |
| 3 | 6,8400 | 6,4675 | 12 | 12,2200 | 3,6390 |
| 4 | 7,3200 | 6,0454 | 13 | 12,6800 | 3,5090 |
| 5 | 8,0800 | 5,4801 | 14 | 13,8400 | 3,2200 |
| 6 | 8,6600 | 5,1156 | 15 | 14,4200 | 3,0930 |
| 7 | 9, 4600 | 9, 6869 | 16 | 14, 9800 | 2,9799 |
| 8 | 9,6800 | 4,5809 | 17 | 18,1000 | 2,4793 |
| 9 | 11,1600 | 3,9796 | | | |

**Tableau 17: Monohydrate d'idazoxan de forme V**

| **Pic** | **Thêta (degré)** | **D (Å)** | **Pic** | **Thêta (degré)** | **D (Å)** |
|---|---|---|---|---|---|
| 1 | 5,0400 | 8,7677 | 9 | 12,9200 | 3,4449 |
| 2 | 5,8400 | 7,5700 | 10 | 13,7400 | 3,2430 |
| 3 | 7,9400 | 5,5761 | 11 | 13,9400 | 3,1973 |
| 4 | 9,2800 | 4,7765 | 12 | 14,5200 | 3,0722 |
| 5 | 9,4400 | 4,6963 | 13 | 14,8200 | 3,0114 |
| 6 | 10,1200 | 4,3837 | 14 | 15,2800 | 2,9228 |
| 7 | 12,0200 | 3,6986 | 15 | 16,2800 | 2,7477 |
| 8 | 12,5600 | 3,5420 | 16 | 16,7400 | 2,6742 |

**Tableau 18: Monohydrate d'idazoxan de forme VI**

| **Pic** | **Thêta (degrés)** | **D (Å)** | **Pic** | **Thêta (degrés)** | **D (Å)** |
|---|---|---|---|---|---|
| 1 | 5,6150 | 7,8723 | 13 | 13,5150 | 3,2959 |
| 2 | 6,7350 | 6,5678 | 14 | 13,9950 | 3,1850 |
| 3 | 7,5350 | 5,8739 | 15 | 14,5250 | 3,0712 |
| 4 | 9,5250 | 4,6547 | 16 | 14,9350 | 2,9887 |
| 5 | 10,3450 | 4,2893 | 17 | 15,0450 | 2,9673 |
| 6 | 10,6050 | 4,1853 | 18 | 15,1950 | 2,9387 |
| 7 | 11,0350 | 4,0241 | 19 | 16,3450 | 2,7370 |
| 8 | 11,2850 | 3,9361 | 20 | 17,0450 | 2,6278 |
| 9 | 11,5350 | 3,8519 | 21 | 17,2850 | 2,5924 |
| 10 | 12,1150 | 3,6701 | 22 | 17,5750 | 2,5509 |
| 11 | 12,3750 | 3,5941 | 21 | 17,8250 | 2,5163 |
| 12 | 12,9550 | 3,4358 | | | |

Ces résultats reflètent la structure cristalline des polymorphes. Le degré de cristallinité différent des différentes formes examinées a donné une modulation importante des intensités, surtout avec l'intensité sensiblement modifiée des rayons X à 4,02 degrés θ. Les diffractogrammes des formes II, III et IV sont visiblement différents les uns des autres. Par exemple, le diffractogramme de la forme II possède trois pics de diffraction à 5,52, 7,5 et 11,52 degrés θ. Le pic à 4,04 degrés θ, caractéristique de la forme III, n'existe pas dans le diffractogramme de la forme II. La forme III présente un pic de diffraction à 4,70 degrés θ, qui n'existe pas dans le diffractogramme de la forme IV. La forme IV présente deux pics à 8,78 et 8,98 degrés θ, qui n'existent pas dans le diffractogramme de la forme III. Le diffractogramme de la forme V est également unique et facile à distinguer des autres.

### Exemple 7: Analyse thermique différentielle sur les polymorphes I à V de l'idazoxan

L'analyse thermique différentielle a été réalisée sur les polymorphes d'idazoxan de forme I à V obtenus conformément au procédé de la présente invention (exemple 5.2).

Les échantillons ont été placés dans des récipients en aluminium fermés et sertis. Trois échantillons de 4 mg de chacune des formes I à V ont été analysés. Les températures de début de fusion ont été déterminées conformément aux directives de l'IUPAC en mesurant la température à l'intersection de la ligne de base étendue (portion linéaire de la courbe de l'analyse thermique différentielle) avec la tangente de la pente la plus raide du flanc antérieur du pic. Les températures finales correspondent au sommet des pics. Le matériel a été calibré en utilisant l'enthalpie de fusion de l'indium (28,5 J/g) comme valeur de référence. Les échantillons ont été soumis à deux analyses à 10°C/mn de 30 à 240°C et de 150 à 240°C. L'objectif de la seconde analyse était de déterminer avec précision les températures de début et de fin de fusion. A cet effet, quatre déterminations ont été réalisées pour chaque échantillon et la moyenne de ces valeurs, de même que l'erreur relative, ont été calculées au moyen des tables de Student-Fischer (t = 3,182 pour P=0,05). Les enthalpies ont été calculées de la même manière, mais sur la base de cinq déterminations (t=2,776, P=0,05).

Une analyse thermogravimétrique a été réalisée sur 9,888 mg de monohydrate d'idazoxan de forme V de 30 à 400°C, sous azote, à 10°C/mn.

Les figures 7 et 8 représentent des thermogrammes représentatifs d'analyse thermique différentielle (comme mesuré par l'appareil cité ci-dessus) pour les formes I à V de l'idazoxan fabriquées conformément aux procédés de la présente invention.

Aucune transformation solide-solide n'a été observée dans aucun échantillon pendant l'augmentation de température avant la fusion.

Les résultats des échantillons multiples sont présentés dans le tableau 19:

**Tableau 19: Analyse thermique différentielle**

| Polymorphe | Température de début(°C) | Pics de température(°C) |
|---|---|---|
| Forme I | 199,3 ± 0,3 | 207,5 ± 0,2 |
| Forme II | 198,1 ± 0,5 | 203,9 ± 0,4 |
| Forme III | 196,9 ± 0,3 | 203,8 ± 0,5 |
| Forme IV | 200,4 ± 0,3 | 205,3 ± 0,5 |
| Forme V | 201 ± 0,4 | 205,6 ± 0,4 |

Comme en témoignent les données présentées ici, les formes I à V de l'idazoxan présentent des thermogrammes d'analyse thermique différentielle uniques.

Le monohydrate d'idazoxan de forme V présente un pic endothermique indiquant la perte d'une molécule d'eau à 87,5 ± 0,4°C avec une enthalpie de déshydratation de 207,6 ± 5,6 J/g. Un second pic endothermique débute à 201 ± 0,4°C et se termine à 205,6 ± 0,4°C, ce qui correspond au point de fusion de l'échantillon et se trouve dans la plage de décomposition de la molécule. Cette décomposition se manifeste par un pic exothermique après fusion se situant autour de 220°C.

Selon le régime de chauffage, c'est à dire la fréquence du balayage, à laquelle l'analyse thermique différentielle est réalisée, les normes de calibrage utilisées, le calibrage de l'appareil, l'humidité relative et la pureté chimique, les endothermes des différents polymorphes de l'idazoxan analysés peuvent varier. Pour un quelconque échantillon donné, l'endotherme observé peut également différer d'un appareil à l'autre, mais il restera généralement dans les plages définies dans le présent document pourvu que les appareils soient calibrés de façon similaire.

### Exemple 8:

### 8.1. Racémisation de l'idazoxan in vitro

Une asymétrie au niveau de la position C2 de la molécule d'idazoxan donne naissance à deux énantiomères, les formes R(-) et S(+). La labilité du proton dans cette position permet une interconversion spontanée entre les deux formes. Des méthodes relevant de la chimie physiologique, de la biochimie et de la biologie ont été utilisées pour étudier la racémisation de l'idazoxan *in vitro.*

La labilité du proton C2 a été étudiée par spectroscopie par résonance magnétique nucléaire de façon à suivre l'échange proton-deutérium dans un tampon phosphate physiologique (pH 7,4) à 37°C. Environ 50% de l'échange s'est produit à l'issue de 80 minutes et l'échange était pratiquement achevé au bout de 4 heures.

La racémisation des deux énantiomères dans un tampon phosphate (pH 7,4) à 25 et 37°C a été étudiée par chromatographie liquide haute pression. La cinétique de racémisation des deux énantiomères s'est révélée identique, avec une demi-vie de racémisation d'environ 5 heures à 25°C et 1 heure à 37°C.

Les valeurs de liaison des α₂-adrénorécepteurs pour les deux énantiomères ont été évaluées au moyen de tissu cortical de rat. En outre, l'activité biologique a été évaluée par l'inhibition de l'hypothermie induite par le guanabenz chez les souris. Initialement, la forme S(+) a montré une affinité de liaison dix fois supérieure à celle de la forme R(-) et s'est révélée 15 fois plus efficace dans l'antagonisme de l'hypothermie. Toutefois, une pré-incubation dans un tampon à 37°C pendant 1 h 30 pour le test de liaison et pendant 4 heures dans le modèle de comportement, a permis de conclure qu'il n'existait pas de différence perceptible d'activité entre les deux énantiomères, ce qui indique que l'équilibre de la racémisation avait bien été atteint.

### 8.2. Pharmacocinétique des énantiomères de l'idazoxan

Des sujets masculins, jeunes et en bonne santé, ont été répartis au hasard pour recevoir une dose unique de chacune des substances suivantes à une occasion: 20 mg de racémate d'idazoxan (polymorphe de forme I); 10 mg de l'énantiomère R(-); 10 mg de l'énantiomère S(+). Les paramètres pharmacocinétiques pour chaque énantiomère sont présentés ci-dessous dans les tableaux 20 et 21.

**Tableau 20:**

| *Paramètres pharmacocinétiques* de *l'idazoxan R(-) suite à une seule administration du racémate (20 mg) ou de l'énantiomère seul (10 mg)* | | | | | | |
|---|---|---|---|---|---|---|
| Elément reçu | Cₘₐₓ ,(ng/mL) | Tₘₐₓ (heure) | AUC_{0-∞} ng.h/mL | t_{1/2} (heure) | CLₜₒₜ/F (L/h) | V_{d}/F (L) |
| Racémate | 11,9±4,6 | 2,1±0,7 | 58±23 | 3,3±0,4 | 170±76 | 789±315 |
| idazoxan R(-) | 10,2±3,9 | 2,5±0,5 | 43±19 | 3,1±0,6 | 230±92 | 988±293 |
| idazoxan | 1,3±0,7 | 3,7±0,5 | 11±6 | 3,7±0,4 | n.a. | n.a. |

**Tableau 21:**

| *Paramètres pharmacocinétiques de l'idazoxan S(+) suite à une seule administration du racémate (20 mg) ou de l'énantiomère seul (10 mg)* | | | | | | |
|---|---|---|---|---|---|---|
| Elément reçu | Cₘₐₓ (ng/mL) | Tₘₐₓ (heure) | AUC_{0-∞} ng.h/mL | t_{1/2} (heure) | CLₜₒₜ/F (L/h) | V_{d}/F (L) |
| Racémate | 8, 3±3, 6 | 2, 1±0,7 | 44±21 | 3,3±0,4 | 245±136 | 1137±540 |
| idazoxan R(-) | 1,6±1,0 | 4,0±0 | 14±8 | 4,1±0,5 | n.a. | n.a. |
| idazoxan S(+) | 7,2±5,1 | 2,1±0,8 | 27±13 | 7,8±0,5 | 397±205 | 1515±692 |

| | | | | | | |
|---|---|---|---|---|---|---|
| n.a.: Non applicable | | | | | | |

Les résultats indiquent que les formes R(-) et S(+) de l'idazoxan présentent des caractéristiques pharmacocinétiques différentes. L'énantiomère S(+) présente une clairance apparente et une distribution environ 1,5 fois supérieures à l'énantiomère R(-), mais un t_{1/2} similaire, entraînant des niveaux de R(-) plus élevés dans le plasma.

Les deux énantiomères sont convertis l'un en l'autre selon un processus lent et limité (Tₘₐₓ d'environ 4 heures; t_{1/2} de conversion de R(-) en S(+) de 5 heures et de S(+) en R(-) de 4 heures), la formation de R(-) étant légèrement supérieure à celle de S(+). Les caractéristiques pharmacocinétiques de chaque énantiomère, après administration du racémate, combinent les caractéristiques pharmacocinétiques de l'énantiomère déterminées après administration de l'énantiomère isolément, suggérant qu'aucune interaction ne se produit entre les énantiomères lorsqu'ils sont administrés ensemble. Les concentrations en S(+) et R(-) culminent et déclinent à un rythme similaire et R(-) circule en plus grande proportion que S(+). Les trois médicaments administrés ont tous été bien tolérés.

### Exemple 9: Utilisation de l'idazoxan comme médicament pour le traitement de la schizophrénie

L'idazoxan a fait l'objet d'une étude en double-aveugle, avec un placebo comme témoin, chez 17 patients souffrant de schizophrénie ou de troubles schizoaffectifs, considérés comme rebelles au traitement avec les neuroleptiques classiques (R.E. Litman, W.W. Hong et al., J. Clin. Psychopharmacol. Août; 13(4): 264-7 (1993); R.E. Litman, T.P. Su et al., Br. J. Psychiatry. Mai; 168(5): 571-9 (1996)).

Les traitements des patients ont été modifiés de façon à les stabiliser pendant au moins 2 semaines avec du chlorhydrate de fluphénazine, du mésylate de benzotropine étant utilisé pour maîtriser les symptômes extrapyramidaux (l'un des patients a été stabilisé avec de la thioridazine en raison de symptômes extrapyramidaux graves). De l'idazoxan a été ajouté à la dose initiale de 20 mg deux fois par jour et la dose a été progressivement augmentée de 20 mg à chaque fois pendant deux semaines jusqu'à une dose fixe, optimale, avec une dose cible de 120 mg/jour maintenue pendant au moins 4 semaines. On a peu à peu réduit la posologie de l'idazoxan de 20 mg par jour pendant 2 semaines, tandis que les patients se voyaient administrer de la fluphénazine seule pendant au moins 3 semaines. Après une période de sevrage thérapeutique, 12 des patients ont reçu de la clozapine, à une posologie optimale, pendant au moins 5 semaines.

L'addition de l'idazoxan au traitement par la fluphénazine a entraîné une réduction significative des symptômes par rapport à une monothérapie à la fluphénazine. Des améliorations ont été observées au niveau de l'échelle globale d'appréciation psychotique de Bunney-Hamburg, du score total de l'échelle abrégée d'appréciation psychiatrique, des symptômes négatifs de l'échelle abrégée d'appréciation psychiatrique et des symptômes positifs de l'échelle abrégée d'appréciation psychiatrique, y compris en ce qui concerne les pensées inhabituelles et la méfiance paranoïaque. Même si l'amélioration s'est révélée modeste (une réduction de l'ordre de 10 à 15% du score total de l'échelle abrégée d'appréciation psychiatrique), elle s'est révélée d'un niveau comparable à celui obtenu en cas de recours à d'autres médicaments non-neuroleptiques pour augmenter l'effet d'agents neuroleptiques. Les améliorations du score total et des symptômes positifs de l'échelle abrégée d'appréciation psychiatrique sont en corrélation avec les modifications observées au niveau des indicateurs noradrénergiques plasmatiques et urinaires.

En outre, le traitement par l'idazoxan associé à la fluphénazine s'est révélé soutenir la comparaison avec celui par la clozapine seule.

## Revendications

1. Composition pharmaceutique comprenant 5 à 25% d'un sel d'idazoxan ou d'hydrate d'idazoxan ou de ses dérivés, 10 à 40% de cellulose microcristalline, 0,1 à 5% de lubrifiant, 0,1 à 0,5% de silice colloïdale et 25 à 90% de lactose par rapport à la masse totale.

2. Composition pharmaceutique selon la revendication 1, comprenant 5 à 20% d'un sel d'idazoxan ou d'hydrate d'idazoxan ou de ses dérivés, 10 à 40% de cellulose microcristalline, 1 à 5% de lubrifiant, 0,1 à 0,5% de silice colloïdale et 29,5 à 84,8% de lactose par rapport à la masse totale.

3. Composition pharmaceutique selon la revendication 1, dans laquelle le sel est le chlorhydrate.

4. Composition pharmaceutique selon la revendication 1 dans laquelle ledit idazoxan est le polymorphe de forme I **caractérisé par** un spectre de diffraction des rayons X comprenant des pics caractéristiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 et 21,4000 degrés θ.

5. Composition pharmaceutique selon la revendication 1, dans laquelle ledit idazoxan est le polymorphe de forme I **caractérisé par** un spectre de diffraction des rayons X comprenant des pics caractéristiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 et 21,4000 degrés θ, et manquant d'au moins un pic à environ 4,7400, 5,7200, 8,9200, 16,8600 ou 18,9000 degrés θ.

6. Composition pharmaceutique selon la revendication 1 dans laquelle ledit polymorphe de forme I est **caractérisé par** un thermogramme d'analyse thermique différentielle présentant une valeur maximale unique à environ 207,5 ± 0,2.

7. Composition pharmaceutique selon la revendication 1 dans laquelle ledit idazoxan est le polymorphe de forme I **caractérisé par** un spectre de diffraction des rayons X comprenant des pics caractéristiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 et 21,4000 degrés θ et par un thermogramme d'analyse thermique différentielle présentant une valeur maximale unique à environ 207,5 ± 0,2.

8. Composition pharmaceutique selon la revendication 1 dans laquelle ledit idazoxan est le polymorphe de forme II **caractérisé par** un spectre de diffraction des rayons X comprenant des pics caractéristiques à environ 4,7400, 5,7200, 6,6800, 7,5000, 8,9200, 9,9600, 11,5200, 12,3000, 12,9400, 13,5400, 14,3000, 15,6800, 16,8600 et 18,9000 degrés θ.

9. Composition pharmaceutique selon la revendication 1 dans laquelle ledit polymorphe de forme II est **caractérisé par** un thermogramme d'analyse thermique différentielle présentant une valeur maximale unique à environ 203,9 ± 0,4.

10. Composition pharmaceutique selon la revendication 1 dans laquelle ledit idazoxan est le polymorphe de forme II **caractérisé par** un spectre de diffraction des rayons X comprenant dés pics caractéristiques à environ 4,7400, 5,7200, 6,6800, 7,5000, 8,9200, 9,9600, 11,5200, 12,3000, 12,9400, 13,5400, 14,3000, 15,6800, 16,8600 et 18,9000 degrés θ et par un thermogramme d'analyse thermique différentielle présentant une valeur maximale unique à environ 203,9 ± 0,4.

11. Composition pharmaceutique selon la revendication 1 dans laquelle ledit idazoxan est le polymorphe de forme III **caractérisé par** un spectre de diffraction des rayons X comprenant des pics caractéristiques à environ 4,0400, 4,7000, 5,7400, 6, 6200, 6, 9200, 7,4600, 8,0400, 8,7800, 8,9800, 9,9800, 10, 8200, 11,4600, 11,6400, 12,3200, 12,9400, 13,5400, 14, 2400, 15,0600, 15,6200 et 16,8400 degrés θ.

12. Composition pharmaceutique selon la revendication 1 dans laquelle ledit polymorphe de forme III est **caractérisé par** un thermogramme d'analyse thermique différentielle présentant une valeur maximale unique à environ 203,8 ± 0,5.

13. Composition pharmaceutique selon la revendication 1 dans laquelle ledit idazoxan est le polymorphe de forme III **caractérisé par** un spectre de diffraction des rayons X comprenant des pics caractéristiques à environ 4,0400, 4,7000, 5,7400, 6,6200, 6,9200, 7,4600, 8,0400, 8,7800, 8,9800, 9,9800, 10,8200, 11,4600, 11, 6400, 12,3200, 12,9400, 13,5400, 14,2400, 15,0600, 15,6200 et 16,8400 degrés θ et par un thermogramme d'analyse thermique différentielle présentant une valeur maximale unique à environ 203,8 ± 0,5.

14. Composition pharmaceutique selon la revendication 1 dans laquelle ledit idazoxan,est le polymorphe de forme IV **caractérisé par** un spectre de diffraction des rayons X comprenant des pics caractéristiques à environ 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 et 18,1000 degrés θ.

15. Composition pharmaceutique selon la revendication 1 dans laquelle ledit idazoxan est le polymorphe de forme IV **caractérisé par** un spectre de diffraction des rayons X comprenant des pics caractéristiques à environ 4,8000, 5,9000, 6,8400, 7,3200, 8,0800, 8,6600, 9,4600, 9,6800, 11,1600, 11,4000, 11, 9000, 12,2200, 12, 6800, 13,8400, 14,4200, 14,9800 et 18,1000 degrés θ et manquant d'au moins un pic à environ 6,6800, 13,5400, 15,6800, 16,8600 ou 18,9000 degrés θ.

16. Composition pharmaceutique selon la revendication 1 dans laquelle ledit polymorphe de forme IV est **caractérisé par** un thermogramme d'analyse thermique différentielle présentant une valeur maximale unique à environ 205,3 ± 0,5.

17. Composition pharmaceutique selon la revendication 1 dans laquelle ledit idazoxan est le polymorphe de forme IV **caractérisé par** un spectre de diffraction des rayons X comprenant des pics caractéristiques à environ 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 et 18,1000 degrés θ et par un thermogramme d'analyse thermique différentielle présentant une valeur maximale unique à environ 205,3 ± 0,5.

18. Composition pharmaceutique selon la revendication 1 dans laquelle ledit monohydrate d'idazoxan est le polymorphe de forme V **caractérisé par** un spectre de diffraction des rayons X comprenant des pics caractéristiques à environ 5,0400, 5,8400, 7,9400, 9,2800, 9,9400, 10,1200, 12,0200, 12,5600, 12,9200, 13,7400, 13,9400, 14,5200, 14,8200, 15,2800, 16,2800 et 16,7400 degrés θ.

19. Composition pharmaceutique selon la revendication 1 dans laquelle ledit monohydrate d'idazoxan est le polymorphe de forme V **caractérisé par** un spectre de diffraction des rayons X comprenant des pics caractéristiques à environ 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 et 16,7400 degrés θ et manquant d'au moins un pic à environ 4,7400, 6,6800, 7,5000, 8,9200, 11,5200, 14,3000, 15,6800 ou 18,9000 degrés θ.

20. Composition pharmaceutique selon la revendication 1 dans laquelle ledit polymorphe de monohydrate d'idazoxan de forme V est **caractérisé par** un thermogramme d'analyse thermique différentielle présentant une valeur maximale unique à environ 205,6 ± 0,4.

21. Composition pharmaceutique selon la revendication 1 dans laquelle ledit monohydrate d'idazoxan est le polymorphe de forme V **caractérisé par** un spectre de diffraction des rayons X comprenant des pics caractéristiques à environ 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 et 16,7400 degrés θ et par un thermogramme d'analyse thermique différentielle présentant une valeur maximale unique à environ 205,6 ± 0,4.

22. Composition pharmaceutique selon la revendication 1 dans laquelle ledit monohydrate d'idazoxan est le polymorphe de forme VI **caractérisé par** un spectre de diffraction des rayons X comprenant des pics caractéristiques à environ 5,6150, 6,7350, 7,5350, 9,5250, 10,3450, 10,6050, 11,0350, 11,2850, 11,5350, 12,1150, 12, 3750, 12,9550, 13,5150, 13,9950, 14,5250, 14, 9350, 15,0450, 15,1950, 16,3450, 17,0450, 17, 2850, 17,5750 et 17,8250 degrés θ.

23. Composition pharmaceutique selon la revendication 1
dans laquelle le lubrifiant est le béhénate de glycérol.

24. Composition pharmaceutique selon la revendication 1 se présentant sous une forme adaptée à l'administration orale.

25. Comprimés contenant une composition pharmaceutique selon l'une quelconque des revendications 1 à 24.

26. Comprimés selon la revendication 25, **caractérisés en ce qu'**ils possèdent une masse comprise entre 50 et 1 000 mg, de préférence entre 100 et 600 mg.

27. Comprimés selon la revendication 25, **caractérisés en ce qu'**ils se présentent dans un conditionnement étanche.

28. Comprimés selon la revendication 27, **caractérisés en ce que** le conditionnement étanche à la vapeur d'eau est constitué par un pilulier en polypropylène ou en polyéthylène haute densité, par un sachet en aluminium et préférentiellement par un blister tout aluminium.

29. Procédé de fabrication d'un comprimé selon la revendication 25, comprenant une étape de compression directe d'un mélange de poudre.

30. Procédé de fabrication d'un comprimé selon la revendication 29 **caractérisé en ce que** ladite compression est précédée d'une étape de granulation sèche.

31. Procédé de fabrication d'un comprimé selon la revendication 30 dans lequel le sel ou l'hydrate d'idazoxan ou de ses dérivés a une granulométrie exprimée par son diamètre moyen compris entre 50 et 250 microns.

32. Procédé de fabrication d'un comprimé selon la revendication 30 dans lequel le sel ou l'hydrate d'idazoxan ou de ses dérivés a une granulométrie moyenne comprise préférentiellement entre 75 et 150 microns et plus particulièrement voisine de 100 à 125 microns.

33. Procédé de fabrication d'un comprimé selon la revendication 29 dans lequel le sel ou l'hydrate d'idazoxan ou de ses dérivés a une densité vrac comprise entre 0,4 et 0,8 et préférentiellement comprise entre 0,5 et 0,7 et de manière encore plus préférée proche de 0,6.

34. Utilisation d'une composition selon l'une quelconque des revendications 1 à 24 ou d'un comprimé selon l'une quelconque des revendications 25 à 28 pour la préparation d'un médicament destiné au traitement et/ou à la prévention d'une pathologie sélectionnée dans le groupe composé de la dépression, de la maladie de Parkinson et des désordres psychotiques sévères, choisis dans le groupe comprenant la schizophrénie et les maladies schizo-affectives.

35. Utilisation d'une composition selon l'une quelconque des revendications 1 à 24 ou d'un comprimé selon l'une quelconque des revendications 25 à 28 pour la préparation d'un médicament destiné au traitement et/ou à la prévention des désordres psychotiques sévères, choisis dans le groupe comprenant la schizophrénie et les maladies schizo-affectives, en association avec un neuroleptique antipsychotique atypique possédant une plus grande affinité antagoniste pour le récepteur D₂ que pour le récepteur alpha-2-noradrénergique, pour une administration simultanée, séparée ou étalée dans le temps.

36. Utilisation selon la revendication 35, **caractérisée en ce que** ledit neuroleptique atypique est choisi parmi l'olanzapine, la quétiapine, la rispéridone, la sertindole ou la ziprasidone.

37. Polymorphe d'idazoxan de forme I dans lequel le spectre de diffraction des rayons X comprend des pics spécifiques à environ 4,0200, 6,6400, 6,9000, 7,0800, 8,0800, 9,0000, 9,9600, 10,8400, 11, 7200, 12,1400, 12,3800, 12,9800, 13,3000, 13,5200, 14,9000, 15,0600, 15,2400 et 21,4000 degrés θ.

38. Polymorphe d'idazoxan de forme I dans lequel le spectre de diffraction des rayons X comprend des pics spécifiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 et 21,4000 degrés θ et manquant d'au moins un pic à environ 4,7900, 5,7200, 8,9200, 16,8600 ou 18,9000 degrés θ.

39. Polymorphe d'idazoxan de forme I dans lequel le thermogramme d'analyse thermique différentielle présente une valeur maximale unique à environ 207,5 ± 0,2.

40. Polymorphe d'idazoxan de forme I dans lequel le spectre de diffraction des rayons X comprend des pics spécifiques à environ 4,0200, 6,6400, 6,9000, 7,0800, 8,0800, 9,0000, 9,9600, 10,8400, 11,7200, 12,1400, 12,3800, 12,9800, 13,3000, 13,5200, 19,9000, 15,0600, 15,2400 et 21,4000 degrés θ et dans lequel le thermogramme d'analyse thermique différentielle présente une valeur maximale unique à environ 207,5 ± 0,2.

41. Polymorphe d'idazoxan de forme II dans lequel le spectre de diffraction de rayons X comprend des pics caractéristiques à environ 4,7400, 5,7200, 6,6800, 7,5000, 8,9200, 9,9600, 11,5200, 12,3000, 12,9400, 13,5400, 14,3000, 15,6800, 16,8600, 18,9000 degré θ.

42. Polymorphe d'idazoxan de forme II dans lequel le thermogramme d'analyse thermique différentielle présenté une valeur maximale unique à environ 203,9 ± 04.

43. Polymorphe d'idazoxan de forme II dans lequel le spectre de diffraction de rayons X comprend des pics caractéristiques à environ 4,7400; 5,7200, 6,6800, 7, 5000,. 8,9200, 9,9600, 11,5200, 12,3000, 12,9400, 13,5400, 14,3000, 15,6800, 16,8600, 18,9000 degré θ et dans lequel le thermogramme d'analyse thermique différentielle présente une valeur maximale unique à environ 203,9 ± 04.

44. Polymorphe d'idazoxan de forme III dans lequel le spectre de diffraction des rayons X comprend des pics caractéristiques à environ 4,0400, 4,7000, 5,7400, 6,6200, 6,9200, 7,4600, 8,0400, 8,7800, 8,9800, 9,9800, 10,8200, 11,4600, 11, 6400, 12,3200, 12,9400, 13,5400, 14,2400, 15,0600, 15,6200 et 16,8400 degrés θ.

45. Polymorphe d'idazoxan de forme III dans lequel le thermogramme d'analyse thermique différentielle présente une valeur maximale unique à environ 203,8 ± 0,5.

46. Polymorphe d'idazoxan de forme III dans lequel le spectre de diffraction des rayons X comprend des pics caractéristiques à environ 4,0400, 4,7000, 5,7400, 6,6200, 6,9200, 7,4600, 8,0400, 8,7800, 8,9800, 9,9800, 10,8200, 11,4600, 11,6400, 12,3200, 12,9400, 13,5400, 14,2400, 15,0600, 15,6200 et 16,8400 degrés θ et dans lequel le thermogramme d'analyse thermique différentielle présente une valeur maximale unique à environ 203,8 ± 0,5.

47. Polymorphe d'idazoxan de forme IV dans lequel le spectre de diffraction des rayons X comprend des pics caractéristiques à environ 4,8000, 5,9000, 6,8400, 7,3200, 8,0800, 8,6600, 9,4600, 9,6800, 11,1600, 11,4000, 11, 9000, 12,2200, 12,6800, 13,8400, 14,4200, 14,9800 et 18,1000 degrés θ.

48. Polymorphe d'idazoxan de forme IV dans lequel le spectre de diffraction des rayons X comprend des pics caractéristiques à environ 4,8000, 5,9000, 6,8400, 7,3200, 8,0800, 8,6600, 9,4600, 9,6800, 11, 1600, 11, 4000, 11,9000, 12,2200, 12,6800, 13,8400, 14,4200, 14,9800 et 18,1000 degrés θ et manquant d'au moins un pic à environ 6,6800, 13,5400, 15,6800, 16,8600 ou 18,9000 degrés θ.

49. Polymorphe d'idazoxan de forme IV dans lequel le thermogramme d'analyse thermique différentielle présente une valeur maximale unique à environ 205,3 ± 0,5.

50. Polymorphe d'idazoxan de forme IV dans lequel le spectre de diffraction des rayons X comprend des pics caractéristiques à environ 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 et 18,1000 degrés θ et manquant d'au moins un pic à environ 6,6800, 13,5400, 15,6800, 16,8600 ou 18,9000 degrés θ et dans lequel le thermogramme d'analyse thermique différentielle présente une valeur maximale unique à environ 205,3 ± 0,5.

51. Polymorphe d'idazoxan de forme V dans lequel le spectre de diffraction des rayons X comprend des pics caractéristiques à environ 5,0400, 5,8400, 7,9400, 9, 2800, . 9, 4400, 10,1200, 12,0200, 12,5600, 12,9200, 13, 7400, 13,9400, 14,5200, 14,8200, 15,2800, 16,2800 et 16,7400 degrés θ.

52. Polymorphe d'idazoxan de forme V dans lequel le spectre de diffraction des rayons X comprend des pics caractéristiques à environ 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 et 16,7400 degrés θ et manquant d'au moins un pic à environ 4,7400, 6,6800, 7,5000, 8,9200, 11,5200, 14,3000, 15,6800 ou 18,9000 degrés θ.

53. Polymorphe d'idazoxan de forme V dans lequel le thermogramme d'analyse thermique différentielle présente une valeur maximale unique à environ 205,6 ± 0,4.

54. Polymorphe d'idazoxan de forme V dans lequel le spectre de diffraction des rayons X comprend des pics caractéristiques à environ 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 et 16,7400 degrés θ et dans lequel le thermogramme d'analyse thermique différentielle présente une valeur maximale unique à environ 205,6 ± 0,4.

55. Polymorphe d'idazoxan de forme VI dans lequel le spectre de diffraction des rayons X comprend des pics caractéristiques à environ 5,6150; 6,7350; 7,5350; 9, 5250: 10,3450; 10,6050; 11, 0350; 11,2850; 11,5350; 12,1150; 12,3750; 12,9550; 13,5150; 13,9950; 14,5250; 14, 9350; 15,0450; 15,1950; 16,3450; 17,0450; 17,2850; 17,5750 et 17,8250 degrés θ.

56. Polymorphe d'idazoxan de forme I produit par un procédé comprenant les étapes suivantes: (i) réaction du catéchol et du 2-chloroacrylonitrile en présence de carbonate de potassium pulvérisé de bromure de tétrabutylammonium comme catalyseur, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, et (v) recristallisation du chlorhydrate d'idazoxan dans de l'éthanol.

57. Polymorphe d'idazoxan de forme I selon la revendication 55, **caractérisé par** un thermogramme d'analyse thermique différentielle ayant une valeur maximale unique à environ 207,5 ± 0,2.

58. Polymorphe d'idazoxan de forme III produit par un procédé comprenant les étapes de (i) réaction du catéchol et du 2-chloroacrylonitrile en présence de carbonate de potassium pulvérisé et de bromure de tétrabutylammonium comme catalyseur, dans un mélange de toluène et. de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol, et (vi) purification du chlorhydrate d'idazoxan par recristallisation avec de l'éthanol.

59. Polymorphe d'idazoxan de forme III selon la revendication 57, **caractérisé par** un thermogramme d'analyse thermique différentielle ayant une une valeur maximale unique à environ 203,8 ± 0,5.

60. Polymorphe d'idazoxan de forme IV produit par un procédé comprenant les étapes de (i) réaction du catéchol et du 2-chloroacrylonitrile en présence de carbonate de potassium pulvérisé et de bromure de tétrabutylammonium comme catalyseur, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediaminé et d'acide chlorhydrique, (iv) addition d'acide chlorhydrique pour cristalliser et éliminer l'éthylènediamine en excès, (v) recristallisation du chlorhydrate d'idazoxan dans de l'éthanol, et (vi) purification du chlorhydrate d'idazoxan par recristallisation avec du butanol-1.

61. Polymorphe d'idazoxan de forme IV selon la revendication 59, **caractérisé par** un thermogramme d'analyse thermique différentielle ayant une une valeur maximale unique à environ 205,3 ± 0,5.

62. Polymorphe d'idazoxan de forme V produit par un procédé comprenant les étapes de (i) réaction du catéchol et du 2-chloroacrylonitrile en présence de carbonate de potassium pulvérisé et de bromure de tétrabutylammonium comme catalyseur, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol, et (vi) réempâtage en cinq volumes d'un mélange de 80% d'acétone et de 20% d'eau.

63. Polymorphe d'idazoxan de forme V selon la revendication 61, **caractérisé par** un thermogramme d'analyse thermique différentielle ayant une valeur maximale unique à environ 205,6 ± 0,4.

64. Polymorphe d'idazoxan de forme VI produit par un procédé comprenant les étapes de (i) réaction du catéchol et du 2-chloroacrylonitrile en présence de carbonate de potassium pulvérisé et d'un catalyseur, le bromure de tétrabutylammonium, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol sous la forme I, (vi) mettre la forme I en suspension dans l'éthanol à 100° sous agitation constante et à température ambiante pendant 1 à 4 jours, (vii) filtrer sous vide, et (viii) sécher dans un four sous vide, ou (vi') maintenir la forme I en suspension dans l'éthanol à 100° sous constante agitation à haute température, (vii') distiller l'éthanol sous vide jusqu'à évaporation partielle du solvant pour induire la cristallisation, (viii') refroidir la solution à 0°, (ix') filtrer et sécher dans un four.

65. Procédé pour la préparation du polymorphe de chlorhydrate d'idazoxan de forme I comprenant les étapes (i) réaction du catéchol et du 2-chloroacrylonitrile en présence de carbonate de potassium pulvérisé et d'un catalyseur, le bromure de tétrabutylammonium, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, et (v) recristallisation du chlorhydrate d'idazoxan dans de l'éthanol.

66. Procédé pour la préparation des polymorphes de chlorhydrate d'idazoxan comprenant les étapes (i) réaction du catéchol et du 2-chloroacrylonitrile en présence de carbonate de potassium pulvérisé et d'un catalyseur, le bromure de tétrabutylammonium, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol, et (vi) purification du chlorhydrate d'idazoxan par recristallisation avec un solvant.

67. Procédé selon la revendication 66, dans lequel le solvant est de l'éthanol et le polymorphe obtenu est le polymorphe de forme III.

68. Procédé selon la revendication 66, dans lequel le solvant est du butanol-1 et le polymorphe obtenu est le polymorphe de forme IV.

69. Procédé pour la préparation du polymorphe de chlorhydrate d'idazoxan dé forme V comprenant les étapes (i) réaction du catéchol et du 2-chloroacrylonitrile en présence de carbonate de potassium pulvérisé et d'un catalyseur, le bromure de tétrabutylammonium, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol, et (vi) réempâtage en cinq volumes d'un mélange de 80% d'acétone et de 20% d'eau.

70. Procédé pour la préparation du polymorphe du chlorhydrate d'idazoxan de forme VI comprenant les étapes de (i) réaction du catéchol et du 2-chloroacrylonitrile en présence de carbonate de potassium pulvérisé et d'un catalyseur, le bromure de tétrabutylammonium, dans un mélange de toluène et de dioxane, (ii) évaporation du toluène et obtention d'un mélange racémique de 2-cyano-1,4-benzodioxane, (iii) cyclisation du 2-cyano-1,4-benzodioxane en présence d'éthylènediamine et d'acide chlorhydrique, (iv) addition d'acide chlorhydrique pour cristalliser et éliminer l'éthylènediamine en excès, (v) recristallisation du chlorhydrate d'idazoxan dans de l'éthanol sous la forme I, (vi) mettre la forme I en suspension dans l'éthanol à 100° sous agitation constante et à température ambiante pendant 1 à 4 jours, (vii) filtrer sous vide, et (viii) sécher dans un four sous vide, ou (vi') maintenir la forme I en suspension dans l'éthanol à 100° sous constante agitation à haute température, (vii') distiller l'éthanol sous vide jusqu'à évaporation partielle du solvant pour induire la cristallisation, (viii') refroidir la solution à 0°, (ix') filtrer et sécher dans un four.

71. Composition pharmaceutique comprenant le chlorhydrate d'idazoxan selon l'une quelconque des revendications 37 à 64, et un excipient pharmaceutiquement acceptable.

72. Composition pharmaceutique selon la revendication 71 sous une forme adaptée à l'administration orale.

73. Composition pharmaceutique selon la revendication 71, sous la forme d'un comprimé.

74. Composition pharmaceutique selon la revendication 71, sous une forme adaptée à l'administration parentérale, intrapéritonéale, intraveineuse, intra-artérielle, transdermale, sublinguale, intramusculaire, rectale, transbuccale, intranasale, liposomale, vaginale ou intra-occulaire ou sous une forme adaptée à la délivrance locale par un cathéter ou un stent.

75. Composition pharmaceutique pour l'administration orale comprenant le chlorhydrate d'idazoxan, un diluant, un agent désintégrant, un lubrifiant et un anti-adhérent.

76. Composition pharmaceutique selon la revendication 75, dans laquelle le diluant est du lactose monohydrate.

77. Composition pharmaceutique selon la revendication 75 dans laquelle l'agent désintégrant est la cellulose microcristalline.

78. Composition pharmaceutique selon la revendication 75, dans laquelle le lubrifiant est le béhénate de glycérol.

79. Composition pharmaceutique selon la revendication 75, dans laquelle l'anti-adhérent est la dioxine de silice colloïdale.

80. Composition pharmaceutique selon la revendication 75, dans laquelle la composition est sous la forme d'un comprimé.

81. Composition pharmaceutique selon la revendication 80, dans laquelle le comprimé est un comprimé de 10 mg.

82. Composition pharmaceutique selon la revendication 80, dans laquelle le comprimé est un comprimé de 20 mg.

83. Composition pharmaceutique selon la revendication 80, dans laquelle le chlorhydrate d'idazoxan comprend du chlorhydrate d'idazoxan selon l'une quelconque des revendications 37 à 64.

84. Composition pharmaceutique pour l'administration orale comprenant:
a. 10% en poids de chlorhydrate d'idazoxan;
b. 61,46% en poids de lactose monohydrate;
c. 26,34% en poids de cellulose microcristalline;
d. 2% en poids de béhénate de glycérol; et
e. 0,2% en poids de dioxine de silicone colloïdale.

85. Composition pharmaceutique selon la revendication 84, dans laquelle la composition est sous la forme d'un comprimé.

86. Composition pharmaceutique selon la revendication 84, dans laquelle le comprimé est un comprimé de 10 mg.

87. Composition pharmaceutique selon la revendication 84, dans laquelle le comprimé est un comprimé de 20 mg.

88. Composition pharmaceutique selon l'une quelconque des revendications 75 à 84, dans laquelle le chlorhydrate d'idazoxan est un énantiomère unique.

89. Composition pharmaceutique selon la revendication 88, dans laquelle l'énantiomère est l'énantiomère S(+).

90. Composition pharmaceutique selon la revendication 88, dans laquelle l'énantiomère est l'énantiomère R(-).

91. Utilisation d'une composition selon l'une quelconque des revendications 71 à 90 pour la préparation d'un médicament destiné au traitement et/ou à la prévention d'une maladie mentale psychotique sévère en combinaison avec un neuroleptique antipsychotique atypique possédant une plus grande affinité antagoniste pour le récepteur D₂ que pour le récepteur alpha-2-noradrénergique dans un excipient pharmaceutiquement acceptable, pour une administration simultanée, séparée ou étalée dans le temps.

## Claims

1. A pharmaceutical composition comprising 5 to 25% of an idazoxan salt or of idazoxan hydrate or its derivatives, 10 to 40% of microcrystalline cellulose, 0.1 to 5% of lubricant, 0.1 to 0.5% of colloidal silica and from 25% to 90% of lactose, with respect to the total mass.

2. The pharmaceutical composition according to claim 1, comprising 5 to 20% of an idazoxan salt or of idazoxan hydrate or its derivatives, 10 to 40% of microcrystalline cellulose, 1 to 5% of lubricant, 0.1 to 0.5% of colloidal silica and from 29.5% to 84.8% of lactose, with respect to the total mass.

3. The pharmaceutical composition according to claim 1, wherein the salt is the hydrochloride.

4. The pharmaceutical composition according to claim 1, wherein said idazoxan is the polymorph form I **characterized by** an X-ray diffraction pattern comprising characteristic peaks at approximately 4.0200, 6.6400, 6.9000, 7.0800, 8.0800, 9.0000, 9.9600, 10.8400, 11.7200, 12.1400, 12.3800, 12.9800, 13.3000, 13.5200, 14.9000, 15.0600, 15.2400 and 21.4000 degrees θ.

5. The pharmaceutical composition according to claim 1, wherein said idazoxan is the polymorph form I **characterized by** an X-ray diffraction pattern comprising characteristic peaks at approximately 4.0200, 6.6400, 6.9000, 7.0800, 8.0800, 9.0000, 9.9600, 10.8400, 11.7200, 12.1400, 12.3800, 12.9800, 13.3000, 13.5200, 14.9000, 15.0600, 15.2400 and 21.4000 degrees θ, and lacking at least one peak at approximately 4.7400, 5.7200, 8.9200, 16.8600 or 18.9000 degrees θ.

6. The pharmaceutical composition according to claim 1, wherein said polymorph form I is **characterized by** a differential thermal analysis thermogram exhibiting a single maximum value at approximately 207.5 ± 0.2.

7. The pharmaceutical composition according to claim 1, wherein said idazoxan is the polymorph form I **characterized by** an X-ray diffraction pattern comprising characteristic peaks at approximately 4.0200, 6.6400, 6.9000, 7.0800, 8.0800, 9.0000, 9.9600, 10.8400, 11.7200, 12.1400, 12.3800, 12.9800, 13.3000, 13.5200, 14.9000, 15.0600, 15.2400 and 21.4000 degrees θ and by a differential thermal analysis thermogram exhibiting a single maximum value at approximately 207.5 ± 0.2.

8. The pharmaceutical composition according to claim 1, wherein said idazoxan is the polymorph form II **characterized by** an X-ray diffraction pattern comprising characteristic peaks at approximately 4.7400, 5.7200, 6.6800, 7.5000, 8.9200, 9.9600, 11.5200, 12.3000, 12.9400, 13.5400, 14.3000, 15.6800, 16.8600 and 18.9000 degrees θ.

9. The pharmaceutical composition according to claim 1, wherein said polymorph form II is **characterized by** a differential thermal analysis thermogram exhibiting a single maximum value at approximately 203.9 ± 0.4.

10. The pharmaceutical composition according to claim 1, wherein said idazoxan is the polymorph form II **characterized by** an X-ray diffraction pattern comprising characteristic peaks at approximately 4.7400, 5.7200, 6.6800, 7.5000, 8.9200, 9.9600, 11.5200, 12.3000, 12.9400, 13.5400, 14.3000, 15.6800, 16.8600 and 18.9000 degrees θ and by a differential thermal analysis thermogram exhibiting a single maximum value at approximately 203.9 ± 0.4.

11. The pharmaceutical composition according to claim 1, wherein said idazoxan is the polymorph form III **characterized by** an X-ray diffraction pattern comprising characteristic peaks at approximately 4.0400, 4.7000, 5.7400, 6.6200, 6.9200, 7.4600, 8.0400, 8.7800, 8.9800, 9.9800, 10.8200, 11.4600, 11.6400, 12.3200, 12.9400, 13.5400, 14.2400, 15.0600, 15.6200 and 16.8400 degrees θ.

12. The pharmaceutical composition according to claim 1, wherein said polymorph form III is **characterized by** a differential thermal analysis thermogram exhibiting a single maximum value at approximately 203.8 ± 0.5.

13. The pharmaceutical composition according to claim 1, wherein said idazoxan is the polymorph form III **characterized by** an X-ray diffraction pattern comprising characteristic peaks at approximately 4.0400, 4.7000, 5.7400, 6.6200, 6.9200, 7.4600, 8.0400, 8.7800, 8.9800, 9.9800, 10.8200, 11.4600, 11.6400, 12.3200, 12.9400, 13.5400, 14.2400, 15.0600, 15.6200 and 16.8400 degrees θ and by a differential thermal analysis thermogram exhibiting a single maximum value at approximately 203.8 ± 0.5.

14. The pharmaceutical composition according to claim 1, wherein said idazoxan is the polymorph form IV **characterized by** an X-ray diffraction pattern comprising characteristic peaks at approximately 4.8000, 5.9000, 6.8400, 7.3200, 8.0800, 8.6600, 9.4600, 9.6800, 11.1600, 11.4000, 11.9000, 12.2200, 12.6800, 13.8400, 14.4200, 14.9800 and 18.1000 degrees θ.

15. The pharmaceutical composition according to claim 1, wherein said idazoxan is the polymorph form IV **characterized by** an X-ray diffraction pattern comprising characteristic peaks at approximately 4.8000, 5.9000, 6.8400, 7.3200, 8.0800, 8.6600, 9.4600, 9.6800, 11.1600, 11.4000, 11.9000, 12.2200, 12.6800, 13.8400, 14.4200, 14.9800 and 18.1000 degrees θ and lacking at least one peak at approximately 6.6800, 13.5400, 15.6800, 16.8600 or 18.9000 degrees θ.

16. The pharmaceutical composition according to claim 1, wherein said polymorph form IV is **characterized by** a differential thermal analysis thermogram exhibiting a single maximum value at approximately 205.3 ± 0.5.

17. The pharmaceutical composition according to claim 1, wherein said idazoxan is the polymorph form IV **characterized by** an X-ray diffraction pattern comprising characteristic peaks at approximately 4.8000, 5.9000, 6.8400, 7.3200, 8.0800, 8.6600, 9.4600, 9.6800, 11.1600, 11.4000, 11.9000, 12.2200, 12.6800, 13.8400, 14.4200, 14.9800 and 18.1000 degrees θ and by a differential thermal analysis thermogram exhibiting a single maximum value at approximately 205.3 ± 0.5.

18. The pharmaceutical composition according to claim 1, wherein said idazoxan monohydrate is the polymorph form V **characterized by** an X-ray diffraction pattern comprising characteristic peaks at approximately 5.0400, 5.8400, 7.9400, 9.2800, 9.4400, 10.1200, 12.0200, 12.5600, 12.9200, 13.7400, 13.9400, 14.5200, 14.8200, 15.2800, 16.2800 and 16.7400 degrees θ.

19. The pharmaceutical composition according to claim 1, wherein said idazoxan monohydrate is the polymorph form V **characterized by** an X-ray diffraction pattern comprising characteristic peaks at approximately 5.0400, 5.8400, 7.9400, 9.2800, 9.4400, 10.1200, 12.0200, 12.5600, 12.9200, 13.7400, 13.9400, 14.5200, 14.8200, 15.2800, 16.2800 and 16.7400 degrees θ and lacking at least one peak at approximately 4.7400, 6.6800, 7.5000, 8.9200, 11.5200, 14.3000, 15.6800 or 18.9000 degrees θ.

20. The pharmaceutical composition according to claim 1, wherein said idazoxan monohydrate is the polymorph form V **characterized by** a differential thermal analysis thermogram exhibiting a single maximum value at approximately 205.6 ± 0.4.

21. The pharmaceutical composition according to claim 1, wherein said idazoxan monohydrate is the polymorph form V **characterized by** an X-ray diffraction pattern comprising characteristic peaks at approximately 5.0400, 5.8400, 7.9400, 9.2800, 9.4400, 10.1200, 12.0200, 12.5600, 12.9200, 13.7400, 13.9400, 14.5200, 14.8200, 15.2800, 16.2800 and 16.7400 degrees θ and by a differential thermal analysis thermogram exhibiting a single maximum value at approximately 205.6 ± 0.4.

22. The pharmaceutical composition according to claim 1, wherein said idazoxan monohydrate is the polymorph form VI **characterized by** an X-ray diffraction pattern comprising characteristic peaks at approximately 5.6150, 6.7350, 7.5350, 9.5250, 10.3450, 10.6050, 11.0350, 11.2850, 11.5350, 12.1150, 12.3750, 12.9550, 13.5150, 13.9950, 14.5250, 14.9350, 15.0450, 15.1950, 16.3450, 17.0450, 17.2850, 17.5750 and 17.8250 degrees θ.

23. The pharmaceutical composition according to claim 1, wherein the lubricant is glyceryl behenate.

24. The pharmaceutical composition according to claim 1, which is provided in a form suitable for oral administration.

25. Tablets .comprising a pharmaceutical composition according to anyone of claims 1 to 24.

26. Tablets according to claim 25, **characterized in that** they have a mass of between 50 and 1,000 mg, preferably between 100 and 600 mg.

27. Tablets according to claim 25, **characterized in that** they are provided in a leaktight packaging.

28. Tablets according to claim 27, **characterized in that** the packaging leaktight to water vapor is composed of a tablet bottle made of polypropylene or of high-density polyethylene, of an aluminum sachet or, and preferably, of an all-aluminum blister pack.

29. A process for the manufacture of a tablet according to claim 25, comprising a stage of direct tableting of a powder mixture.

30. The process for the manufacture of a tablet according to claim 29, **characterized in that** said tableting is preceded by a stage of dry granulation.

31. The process for the manufacture of a tablet according to claim 30, wherein the salt or the hydrate of idazoxan or of its derivatives has a particule size, expressed by its mean diameter, of between 50 and 250 microns.

32. The process for the manufacture of a tablet according to claim 30, wherein the salt or the hydrate of idazoxan or of its derivatives has a mean particule size preferably of between 75 and 150 microns and more particularly in the region of 100 to 125 microns.

33. The process for the manufacture of a tablet according to claim 29, wherein the salt or the hydrate of idazoxan or of its derivatives has a bulk density of between 0.4 and 0.8 and preferably of between 0.5 and 0.7 and more still in the region of 0.6.

34. The use of a composition according to any of claims 1 to 24 or of a tablet according to any of claims 25 to 28 for the preparation of a medicament intended for the treatment and/or the prevention of a pathology selected from the group consisting of depression, Parkinson's disease and severe psychotic disorders selected from the group comprising schizophrenia and schizoaffective disorders.

35. The use of a composition according to any of claims 1 to 24 or of a tablet according to any of claims 25 to 28 for the preparation of a medicament intended for the treatment and/or the prevention of severe psychotic disorders, chosen from the group comprising schizophrenia and schizoaffective disorders, in association with an atypical antipsychotic neuroleptic exhibiting a greater antagonistic affinity for the D₂ receptor than for the alpha-2-noradrenergic receptor, for a simultaneous, separate or sequential administration.

36. The use according to claim 35, wherein said atypical neuroleptic is chosen from olanzapine, quetiapine, risperidone, sertindole or ziprasidone.

37. An idazoxan polymorphic form I wherein the X-ray diffraction pattern comprises specific peaks at approximately 4.0200, 6.6400, 6.9000, 7.0800, 8.0800, 9.0000, 9.9600, 10.8400, 11.7200, 12.1400, 12.3800, 12.9800, 13.3000, 13.5200, 14.9000, 15.0600, 15.2400 and 21.4000 degrees θ.

38. An idazoxan polymorphic form I wherein the X-ray diffraction pattern comprises specific peaks at approximately 4.0200, 6.6400, 6.9000, 7.0800, 8.0800, 9.0000, 9.9600, 10.8400, 11.7200, 12.1400, 12.3800, 12.9800, 13.3000, 13.5200, 14.9000, 15.0600, 15.2400 and 21.4000 degrees θ and lacks at least one peak at approximately 4.7400, 5.7200, 8.9200, 16.8600 or 18.9000 degrees θ.

39. An idazoxan polymorphic form I wherein the differential thermal analysis thermogram exhibits a single maximum value at approximately 207.5 ± 0.2.

40. An idazoxan polymorphic form I wherein the X-ray diffraction pattern comprises specific peaks at approximately 4.0200, 6.6400, 6.9000, 7.0800, 8.0800, 9.0000, 9.9600, 10.8400, 11.7200, 12.1400, 12.3800, 12.9800, 13.3000, 13.5200, 14.9000, 15.0600, 15.2400 and 21.4000 degrees θ and wherein the differential thermal analysis thermogram exhibits a single maximum value at approximately 207.5 ± 0.2.

41. An idazoxan polymorphic form II wherein the X-ray diffraction pattern comprises characteristic peaks at approximately 4.7400, 5.7200, 6.6800, 7.5000, 8.9200, 9.9600, 11.5200, 12.3000, 12.9400, 13.5400, 14.3000, 15.6800, 16.8600 and 18.9000 degrees θ.

42. An idazoxan polymorphic form II wherein the differential thermal analysis thermogram exhibits a single maximum value at approximately 203.9 ± 04.

43. An idazoxan polymorphic form II wherein the X-ray diffraction pattern comprises characteristic peaks at approximately 4.7400, 5.7200, 6.6800, 7.5000, 8.9200, 9.9600, 11.5200, 12.3000, 12.9400, 13.5400, 14.3000, 15.6800, 16.8600 and 18.9000 degrees θ and wherein the differential thermal analysis thermogram exhibits a single maximum value at approximately 203.9 ± 04.

44. An idazoxan polymorphic form III wherein the X-ray diffraction pattern comprises characteristic peaks at approximately 4.0400, 4.7000, 5.7400, 6.6200, 6.9200, 7.4600, 8.0400, 8.7800, 8.9800, 9.9800, 10.8200, 11.4600, 11.6400, 12.3200, 12.9400, 13.5400, 14.2400, 15.0600, 15.6200 and 16.8400 degrees θ.

45. An idazoxan polymorphic form III wherein the differential thermal analysis thermogram exhibits a single maximum value at approximately 203.8 ± 0.5.

46. An idazoxan polymorphic form III wherein the X-ray - diffraction pattern comprises characteristic peaks at approximately 4.0400, 4.7000, 5.7400, 6.6200, 6.9200, 7.4600, 8.0400, 8.7800, 8.9800, 9.9800, 10.8200, 11.4600, 11.6400, 12.3200, 12.9400, 13.5400, 14.2400, 15.0600, 15.6200 and 16.8400 degrees θ and wherein the differential thermal analysis thermogram exhibits a single maximum value at approximately 203.8 ± 0.5.

47. An idazoxan polymorphic form IV wherein the X-ray diffraction pattern comprises characteristic peaks at approximately 4.8000, 5.9000, 6.8400, 7.3200, 8.0800, 8.6600, 9.4600, 9.6800, 11.1600, 11.4000, 11.9000, 12.2200, 12.6800, 13.8400, 14.4200, 14.9800 and 18.1000 degrees θ.

48. An idazoxan polymorphic form IV wherein the X-ray diffraction pattern comprises characteristic peaks at approximately 4.8000, 5.9000, 6.8400, 7.3200, 8.0800, 8.6600, 9.4600, 9.6800, 11.1600, 11.4000, 11.9000, 12.2200, 12.6800, 13.8400, 14.4200, 14.9800 and 18.1000 degrees θ and lacks at least one peak at approximately 6.6800, 13.5400, 15.6800, 16.8600 or 18.9000 degrees θ.

49. An idazoxan polymorphic form IV wherein the differential thermal analysis thermogram exhibits a single maximum value at approximately 205.3 ± 0.5.

50. An idazoxan polymorphic form IV wherein the X-ray diffraction pattern comprises characteristic peaks at approximately 4.8000, 5.9000, 6.8400, 7.3200, 8.0800, 8.6600, 9.4600, 9.6800, 11.1600, 11.4000, 11.9000, 12.2200, 12.6800, 13.8400, 14.4200, 14.9800 and 18.1000 degrees θ and lacks at least one peak at approximately 6.6800, 13.5400, 15.6800, 16.8600 or 18.9000 degrees θ and wherein the differential thermal analysis thermogram exhibits a single maximum value at approximately 205.3 ± 0.5.

51. An idazoxan polymorphic form V wherein the X-ray diffraction pattern comprises characteristic peaks at approximately 5.0400, 5.8400, 7.9400, 9.2800, 9.4400, 10.1200, 12.0200, 12.5600, 12.9200, 13.7400, 13.9400, 14.5200, 14.8200, 15.2800, 16.2800 and 16.7400 degrees θ.

52. An idazoxan polymorphic form V wherein the X-ray diffraction pattern comprises characteristic peaks at approximately 5.0400, 5.8400, 7.9400, 9.2800, 9.4400, 10.1200, 12.0200, 12.5600, 12.9200, 13.7400, 13.9400, 14.5200, 14.8200, 15.2800, 16.2800 and 16.7400 degrees θ and lacks at least one peak at approximately 4.7400, 6.6800, 7.5000, 8.9200, 11.5200, 14.3000, 15.6800 or 18.9000 degrees θ.

53. An idazoxan polymorphic form V wherein the differential thermal analysis thermogram exhibits a single maximum value at approximately 205.6 ± 0.4.

54. An idazoxan polymorphic form V wherein the X-ray diffraction pattern comprises characteristic peaks at approximately 5.0400, 5.8400, 7.9400, 9.2800, 9.4400, 10.1200, 12.0200, 12.5600, 12.9200, 13.7400, 13.9400, 14.5200, 14.8200, 15.2800, 16.2800 and 16.7400 degrees θ and wherein the differential thermal analysis thermogram exhibits a single maximum value at approximately 205.6 ± 0.4.

55. An idazoxan polymorphic form VI wherein the X-ray diffraction pattern comprises characteristic peaks at approximately 5.6150, 6.7350, 7.5350, 9.5250, 10.3450, 10.6050, 11.0350, 11.2850, 11.5350, 12.1150, 12.3750, 12.9550, 13.5150, 13.9950, 14.5250, 14.9350, 15.0450, 15.1950, 16.3450, 17.0450, 17.2850, 17.5750 and 17.8250 degrees θ.

56. An idazoxan polymorphic form I produced by a process comprising the steps of: (i) reacting catechol and 2-chloroacrylonitrile in the presence of ground potassium carbonate and tetra-butylammonium bromide as a catalyst, in a mixture of toluene- and-dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine and (v) recrystallizing idazoxan hydrochloride in ethanol.

57. An idazoxan polymorphic form I according to claim 55, **characterized by** a differential thermal analysis thermogram having a single maximum value at approximately 207.5 ± 0.2.

58. An idazoxan polymorphic form III produced by a process comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol, and (vi) purifying the idazoxan hydrochloride by recrystallizing with ethanol.

59. An idazoxan polymorphic form III according to claim 57, **characterized by** a differential thermal analysis thermogram having a single maximum value at approximately 203.8 ± 0.5.

60. An idazoxan polymorphic form IV produced by a process comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol, and (vi) purifying the idazoxan hydrochloride by recrystallizing with butanol-1.

61. An idazoxan polymorphic form IV according to claim 59, **characterized by** a differential thermal analysis thermogram having a single maximum value at approximately 205.3 ± 0.5.

62. An idazoxan polymorphic form V produced by a process comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol, and (vi) resslurying in five volumes of a mixture of 80% acetone and 20% water.

63. An idazoxan polymorphic form V according to claim 77, **characterized by** a differential thermal analysis thermogram having a single maximum value at approximately 205.6 ± 0.4.

64. An idazoxan polymorphic form VI produced by a process comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol under form I, (vi) keeping form I in suspension in ethanol 100° under constant agitation and at room temperature during 1 to 4 days, (vii) filtrating under vacuum, and (viii) drying in an oven under vacuum, or (vi') keeping form I in suspension in ethanol 100° under constant agitation at high temperature, (vii') distillating ethanol under vacuum until partial solvent evaporation to induce crystallisation, (viii') cooling down the solution at 0°, (ix') filtration and drying in an oven.

65. A process for preparing idazoxan hydrochloride polymorph form I comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, and (v) recrystallizing idazoxan hydrochloride in ethanol.

66. A process for preparing idazoxan hydrochloride polymorphs comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol, and (vi) purifying the idazoxan hydrochloride by recrystallizing with a solvent.

67. The process according to claim 66, wherein the solvent is ethanol and the obtained polymorph is polymorph form III.

68. The process according to claim 66, wherein the solvent is butanol-1 and the obtained polymorph is polymorph form IV.

69. A process for preparing idazoxan hydrochloride polymorph form V comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol, and (vi) resslurying in five volumes of a mixture of 80% acetone and 20% water.

70. A process for preparing idazoxan hydrochloride polymorph form VI comprising the steps of: (i) reacting catechol and 2-chloroacrylonitrile in the presence of ground potassium carbonate and tetra-butylammonium bromide as a catalyst, in a mixture of toluene and dioxane, (ii) evaporating the toluene and obtaining a racemic mixture of 2-cyano-1,4-benzodioxan, (iii) cyclizing 2-cyano-1,4-benzodioxan in the presence of ethylenediamine and hydrochloric acid, (iv) adding hydrochloric acid to crystallize and remove excess ethylenediamine, (v) recrystallizing idazoxan hydrochloride in ethanol under form I, (vi) keeping form I in suspension in ethanol 100° under constant agitation and at room temperature during 1 to 4 days, (vii) filtrating under vacuum, and (viii) drying in an oven under vacuum, or (vi') keeping form I in suspension in ethanol 100° under constant agitation at high temperature, (vii') distillating ethanol under vacuum until partial solvent evaporation to induce crystallisation, (viii') cooling down the solution at 0°, (ix') filtration and drying in an oven.

71. The pharmaceutical composition comprising idazoxan hydrochloride according to anyone of claims 37 to 64 and a pharmaceutically acceptable carrier.

72. The pharmaceutical composition according to claim 71 in a form suitable for oral administration.

73. The pharmaceutical composition according to claim 71 in the form of a tablet.

74. The pharmaceutical composition according to claim 71 in a form suitable for parenteral, intraperitoneal, intravenous, intraarterial, transdermal, sublingual, intramuscular, rectal, transbuccal, intranasal, liposomal, vaginal or intraocular administration, or in a form suitable for local delivery by catheter or stent.

75. A pharmaceutical composition for oral administration comprising idazoxan hydrochloride, a diluent, a disintegrating agent, a lubricant and a glidant.

76. The pharmaceutical composition according to claim 75, wherein the diluent is monohydrate lactose.

77. The pharmaceutical composition according to claim 75 wherein the disintegrating agent is microcrystalline cellulose.

78. The pharmaceutical composition according to claim 75, wherein the lubricant is glyceryl behenate.

79. The pharmaceutical composition according to claim 75, wherein the glidant is colloidal silicon dioxide.

80. The pharmaceutical composition according to claim 75, wherein the composition is in the form of a tablet.

81. The pharmaceutical composition according to claim 80, wherein the tablet is a 10 mg tablet.

82. The pharmaceutical composition according to claim 80, wherein the tablet is a 20 mg tablet.

83. The pharmaceutical composition according to claim 80, wherein the idazoxan hydrochloride comprises idazoxan hydrochloride according to anyone of claims 37 to 64.

84. A pharmaceutical composition for oral administration comprising:
a. 10% by weight of idazoxan hydrochloride;
b. 61.46% by weight of monohydrate lactose;
c. 26.34% by weight of microcrystalline cellulose;
d. 2% by weight of glyceryl behenate; and
e. 0.2% by weight of colloidal silicon dioxide.

85. The pharmaceutical composition according to claim 84, wherein the composition is in the form of a tablet.

86. The pharmaceutical composition according to claim 84, wherein the tablet is a 10 mg tablet.

87. The pharmaceutical composition according to claim 84, wherein the tablet is a 20 mg tablet.

88. The pharmaceutical composition according to anyone of claims 75 to 84, wherein the idazoxan hydrochloride is a single enantiomer.

89. The pharmaceutical composition according to claim 88, wherein the enantiomer is the S(+) enantiomer.

90. The pharmaceutical composition according to claim 88, wherein the enantiomer is the R(-) enantiomer.

91. The use of a composition according to anyone of claims 71 to 90 for the preparation of a medicament intended for the treatment and/or the prevention of a severe psychotic mental illness in combination with an atypical antipsychotic neuroleptic exhibiting a greater antagonist affinity for the D₂ receptor than for the alpha-2-adrenoreceptor, in a pharmaceutically acceptable carrier, for a simultaneous, separate, or sequential administration.

## Patentansprüche

1. Pharmazeutische Zusammensetzung, umfassend 5 bis 25 % eines Salzes von Idazoxan oder ldazoxan-Hydrat oder seiner Derivate, 10 bis 40 % mikrokristalline Cellulose, 0,1 bis 5 % Gleitmittel, 0,1 bis 0,5 % kolloidales Siliciumdioxid und 25 bis 90 % Lactose, bezogen auf die Gesamtmasse.

2. Pharmazeutische Zusammensetzung nach Anspruch 1, umfassend 5 bis 20 % eines Salzes von ldazoxan oder Idazoxan-Hydrat oder seiner Derivate, 10 bis 40 % mikrokristalline Cellulose, 1 bis 5 % Gleitmittel, 0,1 bis 0,5 % kolloidales Siliciumdioxid und 29,5 bis 84,8 % Lactose, bezogen auf die Gesamtmasse.

3. Pharmazeutische Zusammensetzung nach Anspruch 1, in der das Salz das Hydrochlorid ist.

4. Pharmazeutische Zusammensetzung nach Anspruch 1, in der das Idazoxan das Polymorphe der Form I ist, welches durch ein Röntgenbeugungsspektrum charakterisiert ist, das charakteristische Peaks bei etwa 4,0200, 6.6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 und 21,4000 Grad θ umfasst.

5. Pharmazeutische Zusammensetzung nach Anspruch 1, in der das Idazoxan das Polymorphe der Form I ist, welches durch ein Röntgenbeugungsspektrum charakterisiert ist, das charakteristische Peaks bei etwa 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 und 21,4000 Grad θ umfasst und dem mindestens ein Peak bei etwa 4,7400, 5,7200, 8,9200, 16,8600 oder 18,9000 Grad θ fehlt.

6. Pharmazeutische Zusammensetzung nach Anspruch 1, in der das Polymorphe der Form I durch ein Differentialthermoanalyse-Thermogramm charakterisiert ist, das einen einzigen maximalen Wert bei etwa 207,5 ± 0,2 aufweist.

7. Pharmazeutische Zusammensetzung nach Anspruch 1, in der das Idazoxan das Polymorphe der Form I ist, welches durch ein Röntgenbeugungsspektrum, das charakteristische Peaks bei etwa 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 und 21,4000 Grad θ umfasst, und durch ein Differentialthermoanalyse-Thermogramm charakterisiert ist, das einen einzigen maximalen Wert bei 207,5 ± 0,2 aufweist.

8. Pharmazeutische Zusammensetzung nach Anspruch 1, in der das Idazoxan das Polymorphe der Form II ist, welches durch ein Röntgenbeugungsspektrum charakterisiert ist, das charakteristische Peaks bei etwa 4,7400, 5,7200, 6,6800, 7,5000, 8,9200, 9,9600, 11,5200, 12,3000, 12,9400, 13,5400, 14,3000, 15,6800, 16,8600 und 18,9000 Grad θ umfasst.

9. Pharmazeutische Zusammensetzung nach Anspruch 1, in der das Polymorphe der Form II durch ein Differentialthermoanalyse-Thermogramm **gekennzeichnet** ist, das einen einzigen maximalen Wert bei etwa 203,9 ± 0,4 aufweist.

10. Pharmazeutische Zusammensetzung nach Anspruch 1, in der das Idazoxan das Polymorphe der Form II ist, welches durch ein Röntgenbeugungsspektrum, das charakteristische Peaks bei etwa 4,7400, 5,7200, 6,6800, 7,5000, 8,9200, 9,9600, 11,5200, 12,3000, 12,9400, 13,5400, 14,3000, 15,6800, 16,8600 und 18,9000 Grad θ umfasst und durch ein Differentialthermoanalyse Thermogramm charakterisiert ist, das einen einzigen maximalen Wert bei etwa 203,9 ± 0,4 aufweist.

11. Pharmazeutische Zusammensetzung nach Anspruch 1, in der das Idazoxan das Polymorphe der Form III ist, welches durch ein Röntgenbeugungsspektrum charakterisiert ist, das charakteristische Peaks bei etwa 4,0400, 4,7000, 5,7400, 6,6200, 6,9200, 7,4600, 8,0400, 8,7800, 8,9800, 9,9800, 10,8200, 11,4600, 11,6400, 12,3200, 12,9400, 13,5400, 14,2400, 15,0600, 15,6200 und 16,8400 Grad θ umfasst.

12. Pharmazeutische Zusammensetzung nach Anspruch 1, in der das Polymorphe der Form III durch ein Differentialthermoanalyse-Thermogramm charakterisiert ist, das einen einzigen maximalen Wert bei etwa 203,8 ± 0,5 aufweist.

13. Pharmazeutische Zusammensetzung nach Anspruch 1, in der das Idazoxan das Polymorphe der Form III ist, welches durch ein Röntgenbeugungsspektrum, das charakteristische Peaks bei etwa 4,0400, 4,7000, 5,7400, 6,6200, 6,9200, 7,4600, 8,0400, 8,7800, 8,9800, 9,9800, 10,8200, 11,4600, 11,6400, 12,3200, 12,9400, 13,5400, 14,2400, 15,0600, 15,6200 und 16,8400 Grad θ umfasst, und durch ein Differentialthermoanalyse-Thermogramm charakterisiert ist, das einen einzigen maximalen Wert bei etwa 203,8 ± 0,5 aufweist.

14. Pharmazeutische Zusammensetzung nach Anspruch 1, in der das Idazoxan das Polymorphe der Form IV ist, welches durch ein Röntgenbeugungsspektrum **gekennzeichnet** ist, das charakteristische Peaks bei etwa 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 und 18,1000 Grad θ umfasst.

15. Pharmazeutische Zusammensetzung nach Anspruch 1, in der das Idazoxan das Polymorphe der Form IV ist, welches durch ein Röntgenbeugungsspektrum charakterisiert ist, das charakteristische Peaks bei etwa 4,8000, 5,9000, 6,8400, 7,3200, 8,0800, 8,6600, 9,4600, 9,6800, 11,1600, 11,4000. 11,9000, 12,2200, 12,6800, 13,8400, 14,4200, 14,9800 und 18,1000 Grad θ umfasst und dem mindestens ein Peak bei etwa 6,6800, 13,5400, 15,6800, 16,8600 oder 18,9000 Grad θ fehlt.

16. Pharmazeutische Zusammensetzung nach Anspruch 1, in der das Polymorphe der Form IV durch ein Differentialthermoanalyse-Thermogramm charakterisiert ist, das einen einzigen maximalen Wert bei etwa 205,3 ± 0,5 aufweist.

17. Pharmazeutische Zusammensetzung nach Anspruch 1, in der das Idazoxan das Polymorphe der Form IV ist, welches durch ein Röntgenbeugungsspektrum, das charakteristische Peaks bei etwa 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 und 18,1000 Grad θ umfasst, und durch ein Differentialihermoanalyse-Thermogramm charakterisiert ist, das einen einzigen maximalen Wert bei etwa 205,3 ± 0,5 aufweist.

18. Pharmazeutische Zusammensetzung nach Anspruch 1, in der das Idazoxan-Monohydrat das Polymorphe der Form V ist, welches durch ein Röntgenbeugungsspektrum **gekennzeichnet** ist, das charakteristische Peaks bei etwa 5,0400, 5,8400, 7,9400, 9,2800, 9,4400, 10,1200, 12,0200, 12,5600, 12,9200, 13,7400, 13,9400, 14,5200, 14.8200, 15,2800, 16,2800 und 16,7400 Grad θ umfasst.

19. Pharmazeutische Zusammensetzung nach Anspruch 1, in der das Idazoxan-Monohydrat das Polymorphe der Form V ist, welches durch ein Röntgenbeugungsspektrum **gekennzeichnet** ist, das charakteristische Peaks bei etwa 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 und 16,7400 Grad θ umfasst und dem mindestens ein Peak bei etwa 4,7400, 6,6800, 7,5000, 8,9200, 11,5200, 14,3000, 15,6800 oder 18,9000 Grad θ fehlt.

20. Pharmazeutische Zusammensetzung nach Anspruch 1, in der das Polymorphe von Idazoxan-Monohydrat der Form V durch ein Differentialthermoanalyse-Thermogramm **gekennzeichnet** ist, das einen einzigen maximalen Wert bei etwa 205,6 ± 0,4 aufweist.

21. Pharmazeutische Zusammensetzung nach Anspruch 1, in der das Idazoxan-Monohydrat das Polymorphe der Form V ist, welches durch ein Röntgenbeugungsspektrum, das charakteristische Peaks bei etwa 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 und 16,7400 Grad θ umfasst, und durch ein Differentialthermoanalyse-Thermogramm charakterisiert ist, das einen einzigen maximalen Wert bei etwa 205,6 ± 04 aufweist.

22. Pharmazeutische Zusammensetzung nach Anspruch 1, in der das Idazoxan-Monohydrat das Polymorphe der Form VI ist, welches durch ein Röntgenbeugungsspektrum charakterisiert ist, das charakteristische Peaks bei etwa 5,6150, 6,7350, 7,5350, 9,5250, 10,3450, 10,6050, 11,0350, 11,2850, 11, 5350, 12,1150, 12, 3750, 12, 9550, 13, 5150, 13, 9950, 14, 5250, 14, 9350, 15,0450, 15,1950, 16,3450, 17,0450, 17,2850, 17,5750 und 17,8250 Grad θ umfasst.

23. Pharmazeutische Zusammensetzung nach Anspruch 1, in der das Gleitmittel das Behenat von Glycerol ist.

24. Pharmazeutische Zusammensetzung nach Anspruch 1, die in einer Form vorliegt, die an eine orale Verabreichung angepasst ist.

25. Tabletten, enthaltend eine pharmazeutische Zusammensetzung nach irgendeinem der Ansprüche 1 bis 24.

26. Tabletten nach Anspruch 25, **dadurch gekennzeichnet, dass** sie eine Masse zwischen 50 und 1000 mg einschließlich, bevorzugt zwischen 100 und 600 mg einschließlich besitzen.

27. Tabletten nach Anspruch 25, **dadurch gekennzeichnet, dass** sie in einer gasdichten Verpackung vorliegen.

28. Tabletten nach Anspruch 27, **dadurch gekennzeichnet, dass** die gegenüber Wasserdampf dichte Verpackung aus einer Pillenabgabevorrichtung aus Polypropylen oder Polyethylen hoher Dichte, aus einem Beutel aus Aluminium oder bevorzugt einem Blister vollständig aus Aluminium besteht.

29. Verfahren zur Herstellung einer Tablette nach Anspruch 25, umfassend einen Schritt der direkten Kompression einer Pulvermischung.

30. Verfahren zur Herstellung einer Tablette nach Anspruch 29, **dadurch gekennzeichnet, dass** der Kompression ein Schritt der trockenen Granulation vorausgeht.

31. Verfahren zur Herstellung einer Tablette nach Anspruch 30, in dem das Salz oder Hydrat von Idazoxan oder seiner Derivate eine Teilchengröße zwischen 50 und 250 Mikrometer, ausgedrückt als ihr mittlerer Durchmesser, aufweist.

32. Verfahren zur Herstellung einer Tablette nach Anspruch 30, in dem das Salz oder Hydrat von Idazoxan oder seiner Derivate eine mittlere Teilchengröße aufweist, die bevorzugt zwischen 75 und 150 Mikrometer einschließlich und spezieller bei etwa 100 bis etwa 125 Mikrometer liegt.

33. Verfahren zur Herstellung einer Tablette nach Anspruch 29, in dem das Salz oder Hydrat von Idazoxan oder seiner Derivate eine Schüttdichte zwischen 0,4 und 0,8 einschließlich und bevorzugt zwischen 0,5 und 0,7 einschließlich und noch mehr bevorzugt nahe 0,6 aufweist.

34. Verwendung einer Zusammensetzung nach irgendeinem der Ansprüche 1 bis 24 oder einer Tablette nach irgendeinem der Ansprüche 25 bis 28 für die Herstellung eines Medikaments, das zur Behandlung und/oder Verhütung einer Krankheit bestimmt ist, die ausgewählt ist aus der Gruppe umfassend Depression, Parkinson-Krankheit und schwere psychotische Störungen, die ausgewählt sind aus der Gruppe umfassend Schizophrenie und schizoaffektive Krankheiten.

35. Verwendung einer Zusammensetzung nach irgendeinem der Ansprüche 1 bis 24 oder einer Tablette nach irgendeinem der Ansprüche 25 bis 28 für die Herstellung eines Medikaments, das zur Behandlung und/oder Prävention von schweren psychotischen Störungen bestimmt ist, die ausgewählt sind aus der Gruppe umfassend Schizophrenie und schizoaffektive Krankheiten, in Verbindung mit einem atypischen antipsychotischen Neuroleptikum, das eine größere antagonistische Affinität zum Rezeptor D₂ als zum alpha-2-noradrenergen Rezeptor besitzt, für eine gleichzeitige, getrennte oder zeitlich gestaffelte Verabreichung.

36. Verwendung nach Anspruch 35, **dadurch gekennzeichnet, dass** das atypische Neuroleptikum ausgewählt ist aus Olanzapin, Quetiapin, Risperidon, Sertindol oder Ziprasidon.

37. Polymorphes von Idazoxan der Form I, bei dem das Röntgenbeugungsspektrum spezifische Peaks bei etwa 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 und 21,4000 Grad θ umfasst.

38. Polymorphes von Idazoxan der Form 1, bei dem das Röntgenbeugungsspektrum spezifische Peaks bei etwa 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 und 21,4000 Grad θ umfasst und dem mindestens ein Peak bei etwa 4,7400, 5,7200, 8,9200, 16,8600 oder 18,9000 Grad θ fehlt.

39. Polymorphes von ldazoxan der Form I, bei dem das Differentialthermoanalyse-Thermogramm einen einzigen maximalen Wert bei etwa 207,5 ± 0,2 aufweist.

40. Polymorphes von Idazoxan der Form I, bei dem das Röntgenbeugungsspektrum charakteristische Peaks bei etwa 4,0200, 6,6400, 6,9000, 7,0800, 8,0800, 9,0000, 9,9600, 10,8400, 11,7200, 12,1400, 12,3800, 12,9800, 13,3000, 13,5200, 14,9000, 15,0600, 15,2400 und 21,4000 Grad θ umfasst und bei dem das Differentialthermoanalyse-Thermogramm einen einzigen maximalen Wert bei 207,5 ± 0,2 aufweist.

41. Polymorphes von Idazoxan der Form II, bei dem das Röntgenbeugungsspektrum charakteristische Peaks bei etwa 4,7400, 5,7200, 6,6800, 7,5000, 8,9200, 9,9600, 11,5200, 12,3000, 12,9400, 13,5400, 14,3000, 15,6800, 16,8600 und 18,9000 Grad θ umfasst.

42. Polymorphes von Idazoxan der Form II, bei dem das Differentialthermoanalyse-Thermogramm einen einzigen maximalen Wert bei etwa 203,9 ± 0,4 aufweist.

43. Polymorphes von Idazoxan der Form II, bei dem das Röntgenbeugungsspektrum charakteristische Peaks bei etwa 4,7400, 5,7200, 6,6800, 7,5000, 8,9200, 9,9600, 11,5200, 12,3000, 12,9400, 13,5400, 14,3000, 15,6800, 16,8600 und 18,9000 Grad θ umfasst und bei dem das Differentialthermoanalyse-Thermogramm einen einzigen maximalen Wert bei etwa 203.9 ± 0,4 aufweist.

44. Polymorphes von Idazoxan der Form III, bei dem das Röntgenbeugungsspektrum charakteristische Peaks bei etwa 4,0400, 4,7000, 5,7400, 6,6200, 6,9200, 7,4600, 8,0400, 8,7800, 8,9800, 9,9800, 10,8200, 11,4600, 11,6400, 12,3200, 12,9400, 13,5400, 14,2400, 15,0600, 15,6200 und 16,8400 Grad θ umfasst.

45. Polymorphes von Idazoxan der Form III, bei dem das Differentialthermoanalyse-Thermogramm einen einzigen maximalen Wert bei etwa 203,8 ± 0,5 aufweist.

46. Polymorphes von Idazoxan der Form III, bei dem das Röntgenbeugungsspektrum spezifische Peaks bei etwa 4,0400, 4,7000, 5,7400, 6,6200, 6.9200, 7,4600, 8,0400, 8,7800, 8,98009 9,9800, 10,8200, 11.4600, 11,6400, 12,3200, 12,9400, 13,5400, 14,2400, 15,0600, 15,6200 und 16,8400 Grad θ umfasst und bei dem das Differentialthermoanalyse-Thermogramm einen einzigen maximalen Wert bei etwa 203,8 ±0,5 aufweist.

47. Polymorphes von Idazoxan der Form IV, bei dem das Röntgenbeugungsspektrum charakteristische Peaks bei etwa 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 und 18,1000 Grad θ umfasst.

48. Polymorphes von Idazoxan der Form IV, bei dem das Röntgenbeugungsspektrum charakteristische Peaks bei etwa 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 und 18,1000 Grad θ umfasst und dem mindestens ein Peak bei etwa 6,6800, 13,5400, 15,6800, 16,8600 oder 18,9000 Grad θ fehlt.

49. Polymorphes von Idazoxan der Form IV, bei dem das Differentialthermoanalyse-Thermogramm einen einzigen maximalen Wert bei etwa 205,3 ± 0,5 aufweist.

50. Polymorphes von Idazoxan der Form IV, bei dem das Röntgenbeugungsspektrum charakteristische Peaks bei etwa 4,8000, 5,9000, 6,8400, 7,3200, 8,0800, 8,6600, 9,4600, 9,6800, 11,1600, 11,4000, 11,9000, 12,2200, 12,6800, 13,8400, 14,4200, 14,9800 und 18,1000 Grad θ umfasst und dem mindestens ein Peak bei etwa 6,6800, 13,5400, 15,6800, 16,8600 oder 18,9000 Grad θ fehlt und bei dem das Differentialthermoanalyse-Thermogramm einen einzigen maximalen Wert bei etwa 205,3 ± 0,5 aufweist.

51. Polymorphes von Idazoxan der Form V, bei dem das Röntgenbeugungsspektrum charakteristische Peaks bei etwa 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 und 16,7400 Grad θ umfasst.

52. Polymorphes von Idazoxan der Form V, bei dem das Röntgenbeugungsspektrum charakteristische Peaks bei etwa 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 und 16,7400 Grad θ umfasst und dem mindestens ein Peak bei etwa 4,7400, 6,6800, 7,5000, 8,9200, 11,5200, 14,3000, 15,6800 oder 18,9000 Grad θ fehlt.

53. Polymorphes von Idazoxan der Form V, bei dem das Differentialthermoanalyse-Thermogramm einen einzigen maximalen Wert bei etwa 205,6 ± 0,4 aufweist.

54. Polymorphes von Idazoxan der Form V, bei dem das Röntgenbeugungsspektrum charakteristische Peaks bei etwa 5,0400, 5,8400, 7,9400, 9,2800, 9,4400, 10,1200, 12,0200, 12,5600, 12,9200, 13,7400, 13,9400, 14,5200, 14,8200, 15,2800, 16,2800 und 16,7400 Grad θ umfasst und bei dem das Differentiatthermoanatyse-Thermogramm einen einzigen maximalen Wert bei etwa 205,6 ± 04 aufweist.

55. Polymorphes von Idazoxan der Form VI, bei dem das Röntgenbeugungsspektrum charakteristische Peaks bei etwa 5,6150, 6,7350, 7,5350, 9,5250. 10,3450, 10,6050, 11,0350, 11,2850, 11,5350, 12,1150, 12,3750, 12,9550, 13,5150, 13,9950, 14,5250, 14,9350, 15,0450, 15,1950, 16,3450, 17,0450, 17,2850, 17,5750 und 17,8250 Grad θ umfasst.

56. Polymorphes von Idazoxan der Form 1, hergestellt durch ein Verfahren, das die folgenden Schritte umfasst: (i) Umsetzen von Brenzcatechin und 2-Chloracrylnitril in Anwesenheit von pulverförmigem Kaliumcarbonat, Tetrabutylammoniumbromid als Katalysator in einer Mischung von Toluol und Dioxan, (ii) Verdampfen des Toluols und Erhalt einer racemischen Mischung von 2-Cyano-1,4-benzodioxan, (iii) Cyclisierung des 2-Cyano-1,4-benzodioxans in Anwesenheit von Ethylendiamin und Chlorwasserstoffsäure, (iv) Zugabe von Chlorwasserstoffsäure, um das überschüssige Ethylendiamin zu kristallisieren und zu eliminieren, und (v) Umkristallisieren des ldazoxan-Hydrochlorids aus Ethanol.

57. Polymorphes von Idazoxan der Form I gemäß Anspruch 55, **gekennzeichnet durch** ein Differentialthermoanalyse-Thermogramm mit einem einzigen maximalen Wert von etwa 207,5 ± 0,2.

58. Polymorphes von Idazoxan der Form III, hergestellt durch ein Verfahren, welches die Schritte umfasst: (i) Umsetzen von Brenzcatechin und 2-Chloracrylnitril in Anwesenheit von pulverförmigem Kaliumcarbonat und Tetrabutylammoniumbromid als Katalysator in einer Mischung von Toluol und Dioxan, (ii) Verdampfen des Toluols und Erhalt einer racemischen Mischung von 2-Cyano-1,4-benzodioxan, (iii) Cyclisierung des 2-Cjrano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisation des ldazoxan-Hydrochlorids aus Ethanol und (vi) Reinigen des Idazoxan-Hydrochlorids durch Umkristallisation mit Ethanol.

59. Polymorphes von Idazoxan der Form III nach Anspruch 57, das durch ein Differentialthermoanalyse-Thermogramm mit einem einzigen maximalen Wert von etwa 203,8 ± 0,5 charakterisiert ist.

60. Polymorphes von Idazoxan der Form IV, hergestellt durch ein Verfahren, welches die Schritte umfasst: (i) Umsetzen von Brenzcatechin und 2-Chloracrylnitril in Anwesenheit von pulverförmigem Kaliumcarbonat und Tetrabutylammoniumbromid als Katalysator in einer Mischung von Toluol und Dioxan, (ii) Verdampfen des Toluols und Erhalt einer racemischen Mischung von 2-Cyano-1,4-benzodioxan, (iii) Cyclisieren des 2-Cyano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisation des Idazoxan-Hydrochlorids aus Ethanol und (vi) Reinigung des ldazoxan-Hydrochlorids durch Umkristallisation mit Butanol-1.

61. Polymorphes von Idazoxan der Form IV nach Anspruch 59, **gekennzeichnet durch** ein Differentialthermoanalyse-Thermogramm mit einem einzigen maximalen Wert bei etwa 205,3 ± 0,5.

62. Polymorphes von Idazoxan der Form V, hergestellt durch ein Verfahren, das die Schritte umfasst: (i) Umsetzen von Brenzcatechin und 2-Chloracrylnitril in Anwesenheit von pulverförmigem Kaliumcarbonat und Tetrabutylammoniumbromid als Katalysator in einer Mischung von Toluol und Dioxan, (ii) Verdampfen des Toluols und Erhalt einer racemischen Mischung von 2-Cyano-1,4-benzodioxan, (iii) Cyclisieren des 2-Cyano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisation des Idazoxan-Hydrochlorids aus Ethanol und (vi) erneutes Aufnehmen in fünf Volumina einer Mischung von 80 % Aceton und 20 % Wasser.

63. Polymorphes von Idazoxan der Form V nach Anspruch 61, **gekennzeichnet durch** ein Differentialthermoanalyse-Thermogramm mit einem einzigen maximalen Wert von etwa 205,6 ± 0,4.

64. Polymorphes von Idazoxan der Form VI, hergestellt durch ein Verfahren, welches die Schritte umfasst: (i) Umsetzen von Brenzcatechin und 2-Chloracrylnitril in Anwesenheit von pulverförmigem Kaliumcarbonat und einem Katalysator, Tetrabutylammoniumbromid, in einer Mischung von Toluol und Dioxan, (ii) Verdampfen des Toluols und Erhalt einer racemischen Mischung von 2-Cyano-1,4-benzodioxan, (iii) Cyclisieren des 2-Cyano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisation des Idazoxan-Hydrochlorids aus Ethanol in der Form I, (vi) Suspendieren der Form I in Ethanol bei 100 ° unter konstantem Rühren und bei Umgebungstemperatur während 1 bis 4 Tagen, (vii) Abfiltrieren unter Vakuum und (viii) Trocknen in einem Vakuumofen oder (vi') Halten der Form I in Suspension in Ethanol bei 100 ° unter konstantem Rühren bei hoher Temperatur, (vii') Abdestillieren des Ethanols unter Vakuum bis zum teilweisen Verdampfen des Lösungsmittels, um die Kristallisation zu induzieren, (viii') Abkühlen der Lösung auf 0 °, (ix') Abfiltrieren und Trocknen in einem Ofen.

65. Verfahren zur Herstellung des Polymorphen von Idazoxan-Hydrochlorid der Form I, umfassend die Schritte: (i) Umsetzen von Brenzcatechin und 2-Chloracrylnitril in Anwesenheit von pulverförmigem Kaliumcarbonat und einem Katalysator, Tetrabutylammoniumbromid, in einer Mischung von Toluol und Dioxan, (ii) Verdampfen des Toluols und Erhalt einer racemischen Mischung von 2-Cyano-1,4-benzodioxan, (iii) Cyclisierung des 2-Cyano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisieren von Idazoxan-Hydrochlorid aus Ethanol.

66. Verfahren zur Herstellung von Polymorphen von Idazoxan-Hydrochlorid, umfassend die Schritte: (i) Umsetzen von Brenzcatechin und 2-Chloracrylnitril in Anwesenheit von pulverförmigem Kaliumcarbonat und einem Katalysator, Tetrabutylammoniumbromid, in einer Mischung von Toluol und Dioxan, (ii) Verdampfen des Toluols und Erhalt einer racemischen Mischung von 2-Cyano-1,4-benzodioxan, (iii) Cyclisierung des 2-Cyano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisieren von Idazoxan-Hydrochlorid aus Ethanol und (vi) Reinigung von Idazoxan-Hydrochlorid durch Umkristallisation mit einem Lösungsmittel.

67. Verfahren nach Anspruch 66, in dem das Lösungsmittel Ethanol ist und das erhaltene Polymorphe das Polymorphe der Form III ist.

68. Verfahren nach Anspruch 66, in dem das Lösungsmittel Butanol-1 ist und das erhaltene Polymorphe das Polymorphe der Form IV ist.

69. Verfahren zur Herstellung des Polymorphen von ldazoxan-Hydrochlorid der Form V, umfassend die Schritte: (i) Umsetzen von Brenzcatechin und 2-Chloracrylnitril in Anwesenheit von pulverförmigem Kaliumcarbonat und einem Katalysator, Tetrabutylammoniumbromid, in einer Mischung von Toluol und Dioxan, (ii) Verdampfen des Toluols und Erhalt einer racemischen Mischung von 2-Cyano-1,4-benzodioxan; (iii) Cyclisierung von 2-Cyano-1,4-benzodioxan in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisieren des Idazoxan-Hydrochlorids aus Ethanol, (vi) erneutes Aufnehmen in 5 Volumina einer Mischung von 80 % Aceton und 20 % Wasser.

70. Verfahren zur Herstellung des Polymorphen von ldazoxan-Hydrochlorid der Form VI, umfassend die Schritte: (i) Umsetzen von Brenzcatechin und 2-Chloracrylnitril in Anwesenheit von pulverförmigem Kaliumcarbonat und einem Katalysator, Tetrabutylammoniumbromid, in einer Mischung von Toluol und Dioxan, (ii) Verdampfen des Toluols und Erhalt einer racemischen Mischung von 2-Cyano-1,4-benzodioxan, (iii) Cyclisierung des 2-Cyano-1,4-benzodioxans in Anwesenheit von Ethylendiamin und Chlorwasserstoffsäure, (iv) Zugabe von Chlorwasserstoffsäure, um überschüssiges Ethylendiamin zu kristallisieren und zu eliminieren, (v) Umkristallisation des Idazoxan-Hydrochlorids in Ethanol in der Form I, (vi) Suspendieren der Form I in Ethanol bei 100 ° unter konstantem Rühren und bei Umgebungstemperatur während 1 - 4 Tagen, (vii) Abfiltrieren unter Vakuum und (viii) Trocknen in einem Vakuumofen oder (vi') Halten der Form I in Suspension in Ethanol bei 100 ° unter konstantem Rühren bei hoher Temperatur, (vii') Abdestillieren des Ethanols unter Vakuum bis zur partiellen Verdampfung des Lösungsmittels, um die Kristallisation zu induzieren, (viii') Abkühlen der Lösung auf 0 °, (ix') Abfiltrieren und Trocknen in einem Ofen.

71. Pharmazeutische Zusammensetzung, umfassend ldazoxan-Hydrochlorid gemäß irgendeinem der Ansprüche 37 bis 64 und einen pharmazeutisch annehmbaren Hilfsstoff.

72. Pharmazeutische Zusammensetzung nach Anspruch 71 in einer Form, die an eine orale Verabreichung angepasst ist.

73. Pharmazeutische Zusammensetzung nach Anspruch 71 in Form einer Tablette.

74. Pharmazeutische Zusammensetzung nach Anspruch 71 in einer Form, die angepasst ist an die parenterale, intraperitoneale, intravenöse, intraarterielle, transdermale, sublinguale, intramuskuläre, rektale, transbukkale, intranasale, liposomale, vaginale oder intraokulare Verabreichung oder eine Verabreichung in einer Form, die an die lokale Zufuhr durch einen Katheter oder einen Stent angepasst ist.

75. Pharmazeutische Zusammensetzung für die orale Verabreichung, umfassend Idazoxan-Hydrochlorid, ein Verdünnungsmittel, ein Zerfallsmittel, ein Gleitmittel und ein Antihaftmittel.

76. Pharmazeutische Zusammensetzung nach Anspruch 75, in der das Verdünnungsmittel Lactose-Monohydrat ist.

77. Pharmazeutische Zusammensetzung nach Anspruch 75, in der das Zerfallsmittel mikrokristalline Cellulose ist.

78. Pharmazeutische Zusammensetzung nach Anspruch 75, in der das Gleitmittel das Behenat von Glycerol ist.

79. Pharmazeutische Zusammensetzung nach Anspruch 75, in der das Antihaftmittel das Dioxin von kolloidalem Siliciumdioxid ist.

80. Pharmazeutische Zusammensetzung nach Anspruch 75, in der die Zusam-Pnensetzung in Form einer Tablette vorliegt.

81. Pharmazeutische Zusammensetzung nach Anspruch 80, in der die Tablette eine 10 mg-Tablette ist.

82. Pharmazeutische Zusammensetzung nach Anspruch 80, in der die Tablette eine 20 mg-Tablette ist.

83. Pharmazeutische Zusammensetzung nach Anspruch 80, in der das Idazoxan-Hydrochlorid ldazoxan-Hydrochlorid nach irgendeinem der Ansprüche 37 bis 64 umfasst.

84. Pharmazeutische Zusammensetzung für die orale Verabreichung, umfassend.
a. 10 Gew.-% Idazoxan-Hydrochlorid;
b. 61,46 Gew.% Lactose-Monohydrat;
c. 26,34 Gew.-% mikrokristalline Zellulose;
d. 2 Gew.-% des Behenats von Glycerol und
e. 0,2 Gew.-% Dioxin von kolloidalem Silicon.

85. Pharmazeutische Zusammensetzung nach Anspruch 84, in der die Zusammensetzung in Form einer Tablette vorliegt.

86. Pharmazeutische Zusammensetzung nach Anspruch 84, in der die Tablette eine 10 mg-Tablette ist.

87. Pharmazeutische Zusammensetzung nach Anspruch 84, in der die Tablette eine 20 mg-Tablette ist.

88. Pharmazeutische Zusammensetzung nach irgendeinem der Ansprüche 75 bis 84, in der das ldazoxan-Hydrochlorid ein einziges Enantiomer ist.

89. Pharmazeutische Zusammensetzung nach Anspruch 88, in der das Enantiomer das S(+)-Enantiomer ist.

90. Pharmazeutische Zusammensetzung nach Anspruch 88, in der das Enantiomer das R(-)-Enantiomer ist.

91. Verwendung einer Zusammensetzung nach irgendeinem der Ansprüche 71 bis 90 für die Herstellung eines Medikaments, das zur Behandlung und/oder Verhütung einer schweren psychotischen Geisteskrankheit in Kombination mit einem atypischen antipsychotischen Neuroleptikum bestimmt ist, welches eine größere antagonistische Affinität zum D₂-Rezeptor als zum alpha-2-noradrenergen Rezeptor besitzt, in einem pharmazeutisch annehmbaren Träger für eine gleichzeitige, getrennte oder zeitlich gestaffelte Verabreichung.
